# EUROPEAN PATENT APPLICATION

(11) **EP 3 673 920 A1**
(43) Date of publication of application: **01.07.2020**
(21) Application number: 18248138.2
(22) Date of filing: 28.12.2018
(51) Int. Cl.: A61K 47/54, A61K 9/51, A61K 47/62, A61K 47/69

(54) **POLYPHENOL-PEPTIDE CONJUGATES FOR NUCLEAR-TARGETED DELIVERY**

(71) Applicant: Universität Wien, 1010 Vienna (AT)
(72) Inventor: BIALAS, Friedrich, 1090 Wien (AT); DEL FAVERO, Giorgia, 1090 Wien (AT); BECKER, Christian, 1090 Wien (AT); MARKO, Doris, 1090 Wien (AT)
(74) Representative: Vossius & Partner Patentanwälte Rechtsanwälte mbB

(57) **Abstract**

The present invention relates to a polyphenol-peptide conjugate composed of: (i) a peptide comprising the amino acid sequence Arg-Arg-X¹-Leu, wherein X¹ refers to one or two hydrophobic amino acids selected independently from lie, Leu, Val, Phe, Trp, and Met; (ii) at least one polyphenol which is covalently bound to the peptide; and (iii) optionally at least one cargo which, if present, is covalently bound to the peptide. The polyphenol-peptide conjugate can be encapsulated with silica. The invention thus also provides silica particles comprising the polyphenol-peptide conjugate encapsulated by a silica shell. The silica particles allow the efficient cellular uptake and targeted delivery of the polyphenol-peptide conjugate into the cell nucleus, and can therefore advantageously be used for nuclear-targeted gene or drug delivery.

## Description

The present invention relates to a polyphenol-peptide conjugate composed of: (i) a peptide comprising the amino acid sequence Arg-Arg-X¹-Leu, wherein X¹ refers to one or two hydrophobic amino acids selected independently from Ile, Leu, Val, Phe, Trp, and Met; (ii) at least one polyphenol which is covalently bound to the peptide; and (iii) optionally at least one cargo which, if present, is covalently bound to the peptide. The polyphenol-peptide conjugate can be encapsulated with silica. The invention thus also provides silica particles comprising the polyphenol-peptide conjugate encapsulated by a silica shell. The silica particles allow the efficient cellular uptake and targeted delivery of the polyphenol-peptide conjugate into the cell nucleus, and can therefore advantageously be used for nuclear-targeted gene or drug delivery.

The efficient delivery of peptides, nucleic acids, pharmacological agents and other biological cargo molecules into cells and specifically into the cell nucleus is highly desirable, e.g., as it allows therapeutic treatments directed at intranuclear targets (Belting M et al., Adv Drug Deliv Rev, 2005, 57(4): 505-27; Deepthi A et al., J Pharm Sci & Res, 2013, 5(2): 48-56).

Delivering biological cargo into cells is a complex process that is difficult to control. Recent studies have relied, *inter alia,* on mesoporous silica nanoparticles as a suitable delivery tool (Watermann A et al., Nanomaterials (Basel), 2017, 7(7) pii: E189; Sapino S et al., Eur J Pharm Biopharm, 2015, 89: 116-25; Murugan C et al., Sci Rep, 2016, 6: 34053; Sarkar A et al., Biochim Biophys Acta, 2016, 1860(10): 2065-75; Liu Z et al., Adv Healthcare Mater, 2017, 6(3): 1601009; AbouAitah K et al., Oncotarget, 2018, 9(41): 26466-90; Xiong L et al., Small, 2015, 11(44): 5919-26; Hemmerich PH et al., PLoS One, 2013, 8(4): e62018; JP-A-2017/095297). However, there is still an urgent and ongoing need for novel and efficient nuclear targeting approaches.

The peptide R5, a naturally occurring 20mer peptide found in the marine diatom *C*. *fusiformis* (Kröger N et al., Science, 1999, 286(5442): 1129-32), generates under biomimetic conditions homogeneous, spherical silica particles (SiPs), and alterations to the peptide structure and sequence, e.g. by adding posttranslational modifications or by shuffling the amino acid sequence, can lead to different particle morphologies (Lechner CC et al., Chem Sci, 2012, 3(12): 3500-4; and Lechner CC et al., J Pept Sci, 2014, 20(2): 152-8). As previously demonstrated, peptide and protein cargo can be efficiently encapsulated into the resulting silica particles without affecting their function (Lechner CC et al., Bioorg Med Chem, 2013, 21(12): 3533-41; and Lechner CC et al., Biomater Sci, 2015, 3(2): 288-97). Moreover, hydrophobic N-terminal modifications of the R5 sequence have been found to support self-assembly of the peptide prior to silica precipitation (Kamalov M et al., Materials Letters, 2018, 212: 114-7; Kamalov M et al., J Pept Sci, 2016, 22: S82). However, none of these silica particles have ever been found to allow a targeted delivery to the nucleus of living cells.

In the context of the present invention, it has surprisingly been found that biomimetic silica particles formed from a covalent conjugate of the R5 peptide (or any other peptide comprising the amino acid sequence Arg-Arg-X¹-Leu, as described further below) with a polyphenol such as quercetin are taken up into cells and are delivered specifically into the nucleus of living eukaryotic cells in a highly efficient manner, as also demonstrated in the appended examples. Remarkably, accumulation in the nucleus is only observed for silica particles comprising the conjugate according to the invention, but not with silica particles comprising the R5 peptide without covalently conjugated polyphenol (see also Example 2). Moreover, the silica particles comprising the conjugate according to the invention have been found to exert no toxic effects on cell viability or DNA integrity, which renders these particles especially well suited for *in vivo* applications, including for use in therapy.

The present invention thus solves the problem of providing a novel and highly efficient means for targeted delivery to the nucleus of eukaryotic cells.

Accordingly, the present invention provides a polyphenol-peptide conjugate comprising (or, preferably, consisting of):
- a peptide, wherein said peptide comprises the amino acid sequence Arg-Arg-X¹-Leu, wherein X¹ refers to one or two hydrophobic amino acids which are each independently selected from lie, Leu, Val, Phe, Trp, and Met;
- at least one polyphenol, wherein each polyphenol is covalently bound to the peptide;
- and optionally at least one cargo, wherein each cargo (if present) is covalently bound to the peptide.

The polyphenol-peptide conjugate according to the invention can further be encapsulated, particularly by a silica shell. The invention thus relates to the polyphenol-peptide conjugate (as described and defined herein), wherein said conjugate is silica-encapsulated.

The present invention likewise provides a silica particle comprising the polyphenol-peptide conjugate (as described and defined herein), wherein said polyphenol-peptide conjugate is encapsulated by a silica shell.

The silica particles are advantageous as they allow to deliver the cargo(s) comprised in the silica-encapsulated polyphenol-peptide conjugate into the nucleus of a living cell in a highly efficient manner, as also demonstrated in Example 2. Accordingly, the present invention relates to the use of a silica particle (as provided herein) as a nuclear-targeted delivery vehicle. Moreover, the invention relates to the use of a silica particle (as provided herein) for nuclear-targeted gene or drug delivery.

The present invention furthermore provides a method of delivering a cargo into the nucleus of a cell, the method comprising contacting the cell with a silica particle (as described and defined herein). It will be understood that the cell to be contacted should be a living eukaryotic cell, and is preferably a living human cell. The method may be an *in vivo* method (e.g., wherein the cell to be contacted is a cell of a human subject/patient, and/or wherein the silica particle is to be administered to a human subject/patient), or the method may be an *in vitro* method (e.g., wherein the cell to be contacted is provided outside a living human or animal body; this includes, in particular, experiments performed with cells in an artificial environment such as an aqueous solution or a culture medium which may be provided, e.g., in a flask, a test tube, a Petri dish, a microtiter plate, etc.).

In particular, the silica particles according to the invention can be used to deliver one or more cargo(s) into the cell nucleus in order to elicit a therapeutic effect or response in the cell (or the corresponding subject/patient). The invention thus also relates to a silica particle, comprising the polyphenol-peptide conjugate which is encapsulated by a silica shell, for use as a medicament (or for use in therapy; e.g., in the treatment of cancer). The invention further relates to the use of the silica particle for the preparation of a medicament, and to the use of the silica particle for the preparation of a medicament for the treatment of a disease or disorder (e.g., cancer). Likewise, the invention provides a pharmaceutical composition comprising the silica particle and optionally a pharmaceutically acceptable excipient.

The present invention provides a novel approach to target biomimetic silica particles to the nucleus of eukaryotic cells without any detectable effects on cell viability. Covalent conjugation of a polyphenol, such as the bioactive food constituent quercetin, to a similarly safe and non-immunogenic peptide, such as the silaffin-based peptide R5, can be used to generate biomimetic silica particles providing an almost ideal cellular shuttle. Cargo can be attached to this shuttle (e.g., via a cysteine residue of the peptide) and can be released within the cell, e.g., via redox-mediated processes. The cargo can be attached via stable linkages to prevent its release before reaching the nucleus. Depending on the desired application, the cargo can also be delivered to the cell nucleus without being released from the polyphenol-peptide conjugate.

The present invention, particularly the polyphenol-peptide conjugate as well as the silica particles provided herein, will be described in more detail in the following. Unless explicitly indicated otherwise, the following description applies individually and specifically to each aspect of the present invention (including, in particular, each of the above-described polyphenol-peptide conjugates, silica particles, methods and uses).

### The peptide comprised in the polyphenol-peptide conjugate

The polyphenol-peptide conjugate according to the present invention comprises a peptide. In particular, it comprises one peptide per one molecule of the conjugate.

The peptide comprised in the polyphenol-peptide conjugate comprises the amino acid sequence Arg-Arg-X¹-Leu. The group X¹ refers to one or two hydrophobic amino acids, preferably to one hydrophobic amino acid, wherein said hydrophobic amino acid(s) is/are each independently selected from Ile, Leu, Val, Phe, Trp, and Met. It will be understood that the amino acids comprised in the amino acid sequence Arg-Arg-X¹-Leu are connected (to one another) via peptide bonds. Preferably, said hydrophobic amino acid(s) is/are each independently selected from Ile, Leu, and Val. More preferably, at least one of said hydrophobic amino acid(s) is Ile. Thus, if X¹ refers to two hydrophobic amino acids, the group X¹ may be, for example, Ile-Ile, lie-Leu, Ile-Val, Leu-Ile, Leu-Leu, Leu-Val, Val-Ile, Val-Leu, or Val-Val; accordingly, the amino acid sequence Arg-Arg-X¹-Leu may be, for example, Arg-Arg-Ile-Ile-Leu, Arg-Arg-Ile-Leu-Leu, Arg-Arg-Ile-Val-Leu, Arg-Arg-Leu-Ile-Leu, Arg-Arg-Leu-Leu-Leu, Arg-Arg-Leu-Val-Leu, Arg-Arg-Val-Ile-Leu, Arg-Arg-Val-Leu-Leu, or Arg-Arg-Val-Val-Leu. It is preferred that X¹ refers to one hydrophobic amino acid which is selected from Ile, Leu, and Val, and even more preferably X¹ is Ile. Accordingly, it is particularly preferred that the peptide (which is comprised in the polyphenol-peptide conjugate) comprises the amino acid sequence Arg-Arg-Ile-Leu.

It is furthermore preferred that the peptide comprises one or more (e.g., one, two, or three) Lys residues. In particular, it is preferred that the amino acid sequence Arg-Arg-X¹-Leu (comprised in the peptide) is preceded by a Lys residue. Thus, even more preferably, the peptide comprises the amino acid sequence Lys-Arg-Arg-Ile-Leu.

The number of amino acids in the peptide (which contains the amino acid sequence Arg-Arg-X¹-Leu, as described herein above) can be varied. The total number of amino acids in the peptide is preferably about 10 to about 40 (e.g., 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40), more preferably about 15 to about 30, even more preferably about 18 to about 22, or yet even more preferably about 20. Thus, it is preferred that the peptide consists of about 10 to about 40 amino acid residues, more preferably of about 15 to about 30 amino acid residues, even more preferably of about 18 to about 22 amino acid residues, and yet even more preferably of about 20 amino acid residues.

It is particularly preferred that the peptide, which is comprised in the polyphenol-peptide conjugate, comprises (or consists of) the amino acid sequence CSSKKSGSYSGSKGSKRRIL. Accordingly, the peptide may comprise the amino acid sequence CSSKKSGSYSGSKGSKRRIL and may be composed of a total of, e.g., 20 to about 40 amino acid residues (e.g., 20 to 35, or 20 to 30, or 20 to 25 amino acid residues). Still more preferably, the peptide consists of 20 amino acid residues and has the amino acid sequence CSSKKSGSYSGSKGSKRRIL. Accordingly, it is particularly preferred that the peptide (which is comprised in the polyphenol-peptide conjugate) is a peptide of SEQ ID NO: 1.

Furthermore, while one or more (or even all) of the amino acid residues of the peptide (including any of the examples of the peptide described in the paragraphs above) may be D-amino acid residues (or while the peptide may be a retro-inverso peptide having only D-amino acid residues), it is preferred that all amino acid residues of the peptide are L-amino acid residues.

### The polyphenol comprised in the polyphenol-peptide conjugate

The polyphenol-peptide conjugate according to the invention contains at least one polyphenol, wherein each polyphenol is covalently bound to the peptide (which is comprised in the conjugate). Thus, a single polyphenol-peptide conjugate may comprise one or more polyphenols (e.g., one, two, three, four, or five polyphenols) which are each covalently bound to the same peptide. Preferably, the conjugate contains one, two or three polyphenols, which are each covalently bound to the peptide. More preferably, the conjugate contains a single (one) polyphenol which is covalently bound to the (one) peptide.

The polyphenol(s) comprised in the polyphenol-peptide conjugate may be the same or different. Preferably, each polyphenol comprised in the polyphenol-peptide conjugate is independently an arene or a heteroarene, wherein said arene or said heteroarene is substituted with two or more -OH (e.g., 2, 3, 4, 5, 6, 7, or 8 groups -OH) and is optionally further substituted with one or more (e.g., one, two, or three) groups R¹, wherein one -OH in each polyphenol is optionally replaced by a group -O-(C₁₋₅ alkylene)-COOH, and wherein each polyphenol is covalently bound to the peptide (preferably via an amide linkage or an ester linkage).

Each R¹ is independently selected from C₁₋₅ alkyl, C₂₋₅ alkenyl, C₂₋₅ alkynyl, -(C₀₋₅ alkylene)-OH, -(C₀₋₅ alkylene)-O(C₁₋₅ alkyl), -(C₀₋₅ alkylene)-O(C₁₋₅ alkylene)-OH, -(C₀₋₅ alkylene)-O(C₁₋₅ alkylene)-O(C₁₋₅ alkyl), -(C₀₋₅ alkylene)-SH, -(C₀₋₅ alkylene)-S(C₁₋₅ alkyl), -(C₀₋₅ alkylene)-S(C₁₋₅ alkylene)-SH, -(C₀₋₅ alkylene)-S(C₁₋₅ alkylene)-S(C₁₋₅ alkyl), -(C₀₋₅ alkylene)-NH₂, -(C₀₋₅ alkylene)-NH(C₁₋₅ alkyl), -(C₀₋₅ alkylene)-N(C₁₋₅ alkyl)(C₁₋₅ alkyl), -(C₀₋₅ alkylene)-halogen, -(C₀₋₅ alkylene)-(C₁₋₅ haloalkyl), -(C₀₋₅ alkylene)-O-(C₁₋₅ haloalkyl), -(C₀₋₅ alkylene)-CN, -(C₀₋₅ alkylene)-NO₂, -(C₀₋₅ alkylene)-CHO, -(C₀₋₅ alkylene)-CO-(C₁₋₅ alkyl), -(C₀₋₅ alkylene)-COOH, -(C₀₋₅ alkylene)-CO-O-(C₁₋₅ alkyl), -(C₀₋₅ alkylene)-O-CO-(C₁₋₅ alkyl), -(C₀₋₅ alkylene)-CO-NH₂, -(C₀₋₅ alkylene)-CO-NH(C₁₋₅ alkyl), -(C₀₋₅ alkylene)-CO-N(C₁₋₅ alkyl)(C₁₋₅ alkyl), -(C₀₋₅ alkylene)-NH-CO-(C₁₋₅ alkyl), -(C₀₋₅ atkylene)-N(C₁₋₅ alkyl)-CO-(C₁₋₅ alkyl), -(C₀₋₅ alkylene)-SO₂-NH₂, -(C₀₋₅ alkylene)-SO₂-NH(C₁₋₅ alkyl), -(C₀₋₅ alkylene)-SO₂-N(C₁₋₅ alkyl)(C₁₋₅ alkyl), -(C₀₋₅ alkylene)-NH-SO₂-(C₁₋₅ alkyl), -(C₀₋₅ alkylene)-N(C₁₋₅ alkyl)-SO₂-(C₁₋₅ alkyl), -(C₀₋₅ alkylene)-SO-(C₁₋₅ alkyl), -(C₀₋₅ alkylene)-SO₂-(C₁₋₅ alkyl), -(C₀₋₅ alkylene)-carbocyclyl, and -(C₀₋₅ alkylene)-heterocyclyl, wherein the carbocyclyl moiety in said -(C₀₋₅ alkylene)-carbocyclyl and the heterocyclyl moiety in said -(C₀₋₅ alkylene)-heterocyclyl are each optionally substituted with one or more (e.g., one, two, or three) R², wherein the C₀₋₅ alkylene moiety in said -(C₀₋₅ alkylene)-carbocyclyl and the C₀₋₅ alkylene moiety in said -(C₀₋₅ alkylene)-heterocyclyl are each optionally substituted with one or more (e.g., one, two, or three) R³, and further wherein one or more (e.g., one, two, or three) -CH₂- units comprised in the C₀₋₅ alkylene moiety in said -(C₀₋₅ alkylene)-carbocyclyl or in the C₀₋₅ alkylene moiety in said -(C₀₋₅ alkylene)-heterocyclyl are each optionally replaced by a group R⁴.

Preferably, each R¹ is independently selected from C₁₋₅ alkyl, -(C₁₋₅ alkylene)-OH, -O(C₁₋₅ alkyl), -(C₁₋₅ alkylene)-O(C₁₋₅ alkyl), -O(C₁₋₅ alkylene)-OH, -(C₁₋₅ alkylene)-O(C₁₋₅ alkylene)-OH, -O(C₁₋₅ alkylene)-O(C₁₋₅ alkyl), -(C₁₋₅ alkylene)-O(C₁₋₅ alkylene)-O(C₁₋₅ alkyl), halogen -(C₁₋₅ alkylene)-halogen, C₁₋₅ haloalkyl, -(C₁₋₅ alkylene)-(C₁₋₅ haloalkyl), -O-(C₁₋₅ haloalkyl), -(C₁₋₅ alkylene)-O-(C₁₋₅ haloalkyl), -CN, -(C₁₋₅ alkylene)-CN, -CHO, -(C₁₋₅ alkylene)-CHO, -CO-(C₁₋₅ alkyl), -(C₁₋₅ alkylene)-CO-(C₁₋₅ alkyl), -COOH, -(C₁₋₅ alkylene)-COOH, -CO-O-(C₁₋₅ alkyl), -(C₁₋₅ alkylene)-CO-O-(C₁₋₅ alkyl), -O-CO-(C₁₋₅ alkyl), -(C₁₋₅ alkylene)-O-CO-(C₁₋₅ alkyl), carbocyclyl (e.g., aryl), -(C₁₋₅ alkylene)-carbocyclyl (e.g., -(C₁₋₅ alkylene)-aryl), heterocyclyl (e.g., heteroaryl), and -(C₁₋₅ alkylene)-heterocyclyl (e.g., -(C₁₋₅ alkylene)-heteroaryl), wherein said carbocyclyl, the carbocyclyl moiety in said -(C₁₋₅ alkylene)-carbocyclyl, said heterocyclyl, and the heterocyclyl moiety in said -(C₁₋₅ alkylene)-heterocyclyl are each optionally substituted with one or more R², wherein the C₁₋₅ alkylene moiety in said -(C₁₋₅ alkylene)-carbocyclyl and the C₁₋₅ alkylene moiety in said -(C₁₋₅ alkylene)-heterocyclyl are each optionally substituted with one or more R³, and further wherein one or more -CH₂- units comprised in the C₁₋₅ alkylene moiety in said -(C₁₋₅ alkylene)-carbocyclyl or in the C₁₋₅ alkylene moiety in said -(C₁₋₅ alkylene)-heterocyclyl are each optionally replaced by a group R⁴. More preferably, each R¹ is independently selected from aryl, -(C₁₋₅ alkylene)-aryl, heteroaryl, and -(C₁₋₅ alkylene)-heteroaryl, wherein said aryl, the aryl moiety in said -(C₁₋₅ alkylene)-aryl, said heteroaryl, and the heteroaryl moiety in said -(C₁₋₅ alkylene)-heteroaryl are each optionally substituted with one or more R², wherein the C₁₋₅ alkylene moiety in said -(C₁₋₅ alkylene)-aryl and the C₁₋₅ alkylene moiety in said -(C₁₋₅ alkylene)-heteroaryl are each optionally substituted with one or more R³, and further wherein one or more -CH₂- units comprised in the C₁₋₅ alkylene moiety in said -(C₁₋₅ alkylene)-aryl or in the C₁₋₅ alkylene moiety in said -(C₁₋₅ alkylene)-heteroaryl are each optionally replaced by a group R⁴

Each R² is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅) alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), and -SO₂-(C₁₋₅ alkyl). Preferably, each R² is independently selected from C₁₋₅ alkyl, -OH, -O(C₁₋₅ alkyl), -O(C₁₋₅ alkylene)-OH, -O(C₁₋₅ alkylene)-O(C₁₋₅ alkyl), halogen, C₁₋₅ haloalkyl, -O-(C₁₋₅ haloalkyl), -CF₃, -CN, -CHO, -CO-(C₁₋₅ alkyl), -COOH, -CO-O-(C₁₋₅ alkyl), and -O-CO-(C₁₋₅ alkyl).

Each R³ is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), and -SO₂-(C₁₋₅ alkyl). Preferably, each R³ is independently selected from -OH, -O(C₁₋₅ alkyl), -O(C₁₋₅ alkylene)-OH, -O(C₁₋₅ alkylene)-O(C₁₋₅ alkyl), halogen, C₁₋₅ haloalkyl, -O-(C₁₋₅ haloalkyl), -CF₃, -CN, -CHO, -CO-(C₁₋₅ alkyl), -COOH, -CO-O-(C₁₋₅ alkyl), and -O-CO-(C₁₋₅ alkyl).

Each R⁴ is independently selected from -O-, -CO-, -CO-O-, -O-CO-, -NH-, -N(C₁₋₅ alkyl)-, -NH-CO-, -N(C₁₋₅ alkyl)-CO-, -CO-NH-, -CO-N(C₁₋₅ alkyl)-, -S-, -SO-, -SO₂-, -SO₂-NH-, -SO₂-N(C₁₋₅ alkyl)-, -NH-SO₂-, and -N(C₁₋₅ alkyl)-SO₂-. Preferably, each R⁴ is independently selected from -O-, -CO-, -CO-O-, and -O-CO-.

As explained above, each polyphenol comprised in the polyphenol-peptide conjugate is covalently bound to the peptide, preferably via an amide linkage or an ester linkage. It will be understood that an amide linkage can be formed from a -COOH group and an -NH₂ group, while an ester linkage can be formed from a -COOH group and an -OH group. More preferably, each polyphenol is independently bound to the peptide via a carboxamide linkage which is formed from a -COOH group in the polyphenol (e.g., from the -COOH group in the aforementioned -O-(C₁₋₅ alkylene)-COOH that replaces one -OH in the polyphenol) and an -NH₂ group in the peptide (e.g., the side-chain -NH₂ group (ε-amino group) of a Lys residue in the peptide, if present; or the N-terminal α-NH₂ group of the peptide), or vice versa (i.e., via a carboxamide linkage formed from a -COOH group in the peptide and an -NH₂ group in the polyphenol), or via an ester linkage which is formed from an -OH group in the polyphenol and a -COOH group in the peptide (e.g., the side-chain COOH group of an Asp or Glu residue in the peptide, if present; or the C-terminal α-COOH group of the peptide), or vice versa (i.e., via an ester linkage formed from a -COOH group in the polyphenol [e.g., from the -COOH group in the aforementioned -O-(C₁₋₅ alkylene)-COOH that replaces one -OH in the polyphenol] and an -OH group in the peptide). Even more preferably, each polyphenol is bound to the peptide via a carboxamide linkage which is formed from a -COOH group in the polyphenol (particularly from the -COOH group in the aforementioned -O-(C₁₋₅ alkylene)-COOH) and an -NH₂ group in the peptide, or vice versa. Still more preferably, one -OH in each polyphenol is replaced by a group -O-(C₁₋₅ alkylene)-COOH, and each polyphenol is covalently bound to the peptide via a carboxamide linkage formed from the -COOH group in said -O-(C₁₋₅ alkylene)-COOH in the polyphenol and an -NH₂ group in the peptide. Furthermore, if the polyphenol-peptide conjugate contains a single polyphenol, it is preferred that one -OH group in said polyphenol is replaced by a group -O-(C₁₋₅ alkylene)-COOH, and that said polyphenol is covalently bound to the peptide via a carboxamide linkage formed from a carboxy group of the polyphenol (particularly from the -COOH of said -O-(C₁₋₅ alkylene)-COOH in the polyphenol) and the N-terminal α-amino group of the peptide.

Preferred examples of the polyphenol(s) comprised in the polyphenol-peptide conjugate include any one of the following substances: quercetin, quercetagetin, fisetin, galangin, gossypetin, herbacetin, kaempferol, morin, myricetin, robinetin, 5-O-methylmyricetin, annulatin, ayanin, axillarin, azaleatin, brickellin, centaureidin, chrysosplenetin, combretol, ermanin, eupatolitin, eupalitin, europetin, isorhamnetin, jaceidin, kaempferide, kumatakenin, laricitrin, natsudaidain, ombuin, pachypodol, patuletin, retusin, mearnsetin, rhamnazin, rhamnetin, santin, spinacetin, syringetin, tamarixetin, noricaritin, karanjachromene, karanjin, pyrocatechol, resorcinol, pyrogallol, phloroglucinol, catechin, epicatechin, catechin gallate, epicatechin gallate, gallocatechin, epigallocatechin, gallocatechin gallate, epigallocatechin gallate, taxifolin, afzelechin, fisetinidol, guibourtinidol, mesquitol, oritin, robinetinidol, meciadanol, or ourateacatechin; wherein one -OH in each of the aforementioned substances is optionally replaced by a group -O-(C₁₋₅ alkylene)-COOH. Each of these polyphenols may be covalently bound to the peptide via an amide linkage or an ester linkage, preferably via a carboxamide linkage formed from a -COOH group in the polyphenol (particularly the -COOH group of the aforementioned -O-(C₁₋₅ alkylene)-COOH) and an -NH₂ group in the peptide or via an ester linkage formed from an -OH group in the polyphenol and a -COOH group in the peptide (particularly the side-chain -COOH group of an Asp or Glu residue in the peptide, if present, or the C-terminal α-COOH group of the peptide), more preferably via a carboxamide linkage formed from a -COOH group in the polyphenol (particularly the -COOH group of the aforementioned -O-(C₁₋₅ alkylene)-COOH) and an -NH₂ group in the peptide.

Accordingly, it is preferred that each polyphenol contained in the polyphenol-peptide conjugate is independently selected from quercetin, quercetagetin, fisetin, galangin, gossypetin, herbacetin, kaempferol, morin, myricetin, robinetin, 5-O-methylmyricetin, annulatin, ayanin, axillarin, azaleatin, brickellin, centaureidin, chrysosplenetin, combretol, ermanin, eupatolitin, eupalitin, europetin, isorhamnetin, jaceidin, kaempferide, kumatakenin, laricitrin, natsudaidain, ombuin, pachypodol, patuletin, retusin, mearnsetin, rhamnazin, rhamnetin, santin, spinacetin, syringetin, tamarixetin, noricaritin, karanjachromene, karanjin, pyrocatechol, resorcinol, pyrogallol, phloroglucinol, catechin, epicatechin, catechin gallate, epicatechin gallate, gallocatechin, epigallocatechin, gallocatechin gallate, epigallocatechin gallate, taxifolin, afzelechin, fisetinidol, guibourtinidol, mesquitol, oritin, robinetinidol, meciadanol, and ourateacatechin, wherein one -OH in each polyphenol is optionally replaced by a group -O-(C₁₋₅ alkylene)-COOH, and wherein each polyphenol is covalently bound to the peptide via an amide linkage or an ester linkage, preferably via a carboxamide linkage formed from a -COOH group in the polyphenol (particularly from the -COOH in the aforementioned group -O-(C₁₋₅ alkylene)-COOH which replaces one -OH in the respective polyphenol) and an -NH₂ group in the peptide.

An even more preferred example of the polyphenol(s) comprised in the polyphenol-peptide conjugate of the invention is quercetin, wherein one -OH in said quercetin (e.g., the 7-OH group of quercetin) is optionally replaced by a group -O-(C₁₋₅ alkylene)-COOH, and wherein said quercetin is covalently bound to the peptide via an amide linkage or an ester linkage, preferably via a carboxamide linkage formed from the -COOH group in said quercetin (in which one -OH is replaced by a group -O-(C₁₋₅ alkylene)-COOH) and an -NH₂ group in the peptide. Accordingly, it is particularly preferred that the polyphenol-peptide conjugate contains at least one quercetin (and optionally one or more further polyphenol(s), such as, e.g., any of the exemplary or preferred polyphenols described herein above), wherein one -OH group in each quercetin is optionally replaced by a group -O-(C₁₋₅ alkylene)-COOH, and wherein each quercetin is covalently bound to the peptide via an amide linkage or an ester linkage, preferably via a carboxamide linkage formed from a -COOH group in quercetin (i.e., from the -COOH in the aforementioned group -O-(C₁₋₅ alkylene)-COOH which replaces one -OH in quercetin) and an -NH₂ group in the peptide. Moreover, the C₁₋₅ alkylene comprised in the aforementioned group -O-(C₁₋₅ alkylene)-COOH is preferably methylene, so that one -OH group of quercetin is preferably replaced by a group -O-CH₂-COOH (and the quercetin is covalently bound to the peptide via a carboxamide linkage formed from said -O-CH₂-COOH group and an -NH₂ group in the peptide).

Yet even more preferably, each polyphenol in the polyphenol-peptide conjugate is 7-(carboxymethoxy)quercetin which is covalently bound to the peptide via a carboxamide linkage formed from the carboxy group of said 7-(carboxymethoxy)quercetin and an amino group of the peptide.

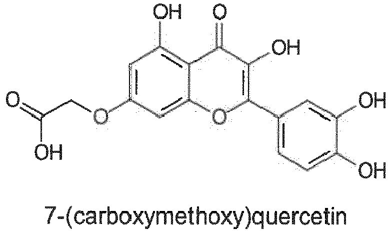

Still more preferably, the polyphenol-peptide conjugate according to the invention contains a single polyphenol, wherein said polyphenol is 7-(carboxymethoxy)quercetin which is covalently bound to the peptide via a carboxamide linkage formed from the carboxy group of said 7-(carboxymethoxy)quercetin and an amino group of the peptide, particularly the N-terminal α-amino group of the peptide.

### The cargo comprised in the polyphenol-peptide conjugate

The polyphenol-peptide conjugate according to the invention optionally comprises at least one cargo. Thus, the conjugate can be provided in "unloaded" state, i.e. without containing any cargo, or it can be provided in "loaded" state, i.e. with one or more cargos comprised in the conjugate. In particular, the conjugate may comprise one or more (e.g., one, two, three, four, five, six, seven, or eight) cargos, which may be the same or different, and which are each covalently bound to the peptide comprised in the conjugate.

The cargo comprised in the conjugate can be delivered specifically to the nucleus of a living cell by contacting the cell with the corresponding silica-encapsulated and cargo-"loaded" conjugate. Thus, it has been found that the silica-encapsulated polyphenol-peptide conjugate according to the present invention is taken up into living cells and localizes not only in the cytosol but also accumulates in the nucleus and, consequently, allows a particularly efficient targeted delivery to the nucleus of a living cell, as also demonstrated in the appended examples.

The polyphenol-peptide conjugate can also be provided in "unloaded" state, i.e. without cargo, but wherein one or more functional groups (e.g., one or more side-chain amino groups, one or more side-chain carboxy groups, the N-terminal α-amino group, and/or the C-terminal α-carboxy group) of the peptide comprised in the conjugate is/are activated. This can facilitate the subsequent attachment of one or more cargo molecules to the unloaded activated conjugate. For example, one or more carboxy groups (such as, e.g., the C-terminal α-carboxy group and/or a side-chain carboxy group of an Asp or Glu residue, if present) of the peptide comprised in the unloaded conjugate can be in the form of an active ester group (e.g., a 1-hydroxybenzotriazole (HOBt) active ester group), an anhydride group (e.g., a propylphosphonic anhydride (T3P) group), or an acyl halide group (e.g., an acyl chloride or acyl fluoride group). Alternatively, the peptide comprised in the unloaded conjugate can also be modified to contain one or more functional groups for "click chemistry" conjugation reactions with corresponding functional groups of the cargo molecule(s), such as, e.g., Cu(I)-catalyzed azide-alkyne cycloaddition (CuAAC), strain-promoted azide-alkyne cycloaddition (SPAAC), oxime click-chemistry ligation, Pictet-Spengler reactions, inverse electron-demand Diels-Alder reactions (DA_{INV}), etc. The activated conjugate can then be reacted with one or more cargo molecules to obtain a corresponding cargo-loaded" conjugate.

In principle, the cargo molecule(s) can be attached to the peptide (which is comprised in the polyphenol-peptide conjugate) via any suitable covalent linkage. For example, the cargo(s) may also be covalently bound to the peptide via a disulfide linkage, which can be formed, e.g., from a mercapto group (-SH) of a cysteine residue comprised in the peptide (if present) and a mercapto group comprised in the cargo (if present).

The cargo(s) comprised in the polyphenol-peptide conjugate is/are not particularly limited and, in principle, any compound of interest can be covalently bound as "cargo" to the peptide comprised in the conjugate. For example, the conjugate may contain at least one cargo, wherein each cargo is independently selected from a peptide/protein, a nucleic acid, a lipid, a sugar, and an active pharmaceutical ingredient (or a therapeutic agent).

The peptide/protein which can be used as cargo for the conjugate according to the invention is preferably a transcription factor. In particular, it may be a transcription factor selected from: a basic domain transcription factor, particularly a basic leucine zipper (bZIP) transcription factor (such as, e.g., AATF, ATF-1, ATF-2, ATF-3, ATF-4, ATF-5, ATF-6, ATF-7, AP-1, c-Fos, FOSB, FOSL1, FOSL2, JDP2, c-Jun, JUNB, JunD, BACH, BACH1, BACH2, BATF, BLZF1, C/EBP, C/EBPα, C/EBPβ, C/EBPγ, C/EBPδ, C/EBPε, C/EBPζ, CREB, CREB1, CREB2, CREB3, CREBL1, CREM, DBP, DDIT3, GABPA, GCN4, HLF, MAF, MAFB, MAFF, MAFG, MAFK, NFE, NFE2, NFEL1, NFEL2, NFEL3, NFIL3, NRL, NRF, NRF1, NRF2, NRF3, or XBP1), a basic helix-loop-helix (bHLH) transcription factor (such as, e.g., ATOH1, AhR, AHRR, ARNT, ASCL1, BHLH, BHLH2, BHLH3, BHLH9, ARNTL, ARNTL1, ARNTL2, CLOCK, EPAS1, FIGLA, HAND, HAND1, HAND2, HES, HES5, HES6, HEY, HEY1, HEY2, HEYL, HES1, HIF, HIF1A, HIF3A, ID, ID1, ID2, ID3, ID4, LYL1, MESP2, MXD4, MYCL1, MYCN, a myogenic regulatory factor (MRF), MyoD, myogenin, MYF5, MYF6, a neurogenin, neurogenin-1, neurogenin-2, neurogenin-3, NeuroD, NeuroD1, NeuroD2, NPAS, NPAS1, NPAS2, NPAS3, OLIG, OLIG1, OLIG2, Pho4, scleraxis, SIM, SIM1, SIM2, TAL, TAL1, TAL2, Twist, or USF1), a basic helix-loop-helix/leucine zipper (bHLH-ZIP) transcription factor (such as, e.g., AP-4, MAX, MXD3, MITF, MNT, MLX, MXI1, Myc, SREBP, SREBP1, or SREBP2), an NF-1 transcription factor (such as, e.g., NFI, NFIA, NFIB, NFIC, NFIX, SMAD, R-SMAD, I-SMAD, SMAD1, SMAD2, SMAD3, SMAD4, SMAD5, SMAD6, SMAD7, SMAD8, or SMAD9), an RF-X transcription factor (such as, e.g., RFX1, RFX2, RFX3, RFX4, RFX5, or RFXANK), or a basic helix-span-helix (bHSH) transcription factor (such as, e.g., AP-2, AP-2α, AP-2β, AP-2γ, AP-2δ, or AP-2ε); a zinc-coordinating/zinc finger DNA-binding domain transcription factor, particularly a Cys₄ zinc finger transcription factor (such as, e.g., a thyroid hormone receptor, thyroid hormone receptor α, thyroid hormone receptor β, CAR, FXR, LXR, LXRα, LXRβ, PPAR, PPARα, PPARβ/δ, PPARγ, PXR, PXR, RAR, RARα, RARβ, RARγ, ROR, RORα, RORβ, RORγ, Rev-ErbA, Rev-ErbAα, Rev-ErbAβ, VDR, COUP-TF, COUP-TFI, COUP-TFII, Ear-2, HNF4, HNF4α, HNF4γ, PNR, RXR, RXRα, RXRβ, RXRγ, testicular receptor, testicular receptor 2, testicular receptor 4, TLX, a steroid hormone receptor, androgen receptor, estrogen receptor, estrogen receptor α, estrogen receptor β, glucocorticoid receptor, mineralocorticoid receptor, progesterone receptor, estrogen related receptor, estrogen related receptor α, estrogen related receptor β, estrogen related receptor γ, NUR, NGFIB, NOR1, NURR1, LRH-1, SF1, GCNF, DAX1, SHP, a GATA transcription factor, GATA1, GATA2, GATA3, GATA4, GATA5, GATA6, MTA, MTA1, MTA2, MTA3, or TRPS1), a Cys₂His₂ zinc finger transcription factor (such as, e.g., TFIIA, TFIIB, TFIID, TFIIE, TFIIE1, TFIIE2, TFIIF, TFIIF1, TFIIF2, TFIIH, TFIIH1, TFIIH2, TFIIH4, TF2I, TF3A, TF3C1, TF3C2, ATBF1, BCL, BCL6, BCL11A, BCL11B, CTCF, E4F1, EGR, EGR1, EGR2, EGR3, EGR4, ERV3, GFI1, GLI1, GLI2, GLI3, REST, GLIS1, GLIS2, YY1, HIC, HIC1, HIC2, HIVEP, HIVEP1, HIVEP2, HIVEP3, IKZF, IKZF1, IKZF2, IKZF3, ILF, ILF2, ILF3, KLF, KLF1, KLF2, KLF3, KLF4, KLF5, KLF6, KLF7, KLF8, KLF9, KLF10, KLF11, KLF12, KLF13, KLF14, KLF15, KLF17, MTF1, MYT1, OSR1, PRDM9, SALL, SALL1, SALL2, SALL3, SALL4, SP, SP1, SP2, SP4, SP7, SP8, TSHZ3, WT1, Zbtb7, Zbtb7A, Zbtb7B, ZBTB, ZBTB11, ZBTB16, ZBTB17, ZBTB20, ZBTB32, ZBTB33, ZBTB40, zinc finger protein 3 (ZNF3), ZNF7, ZNF9, ZNF10, ZNF19, ZNF22, ZNF24, ZNF33B, ZNF34, ZNF35, ZNF41, ZNF43, ZNF44, ZNF51, ZNF74, ZNF143, ZNF146, ZNF148, ZNF165, ZNF202, ZNF217, ZNF219, ZNF238, ZNF239, ZNF259, ZNF267, ZNF268, ZNF281, ZNF295, ZNF300, ZNF318, ZNF330, ZNF346, ZNF350, ZNF365, ZNF366, ZNF384, ZNF423, ZNF451, ZNF452, ZNF471, ZNF593, ZNF638, ZNF644, ZNF649, or ZNF655), a Cys₆ zinc finger transcription factor (such as, e.g., HIVEP1), AIRE, DID01, GRLF1, ING, ING1, ING2, ING4, JARID, JARID1A, JARID1B, JARID1C, JARID1D, JARID2, JMJD1B, or WRKY; a helix-turn-helix domain transcription factor, particularly a homeodomain transcription factor (such as, e.g., ARX, CDX, CDX1, CDX2, CRX, CUTL1, DBX, DBX1, DBX2, DLX, DLX1, DLX2, DLX3, DLX4, DLX5, DLX6, EMX, EMX1, EMX2, EN, EN1, EN2, FHL, FHL1, FHL2, FHL3, HESX1, HHEX, HLX, a homeobox protein, homeobox A1 (Hox-A1), Hox-A2, Hox-A3, Hox-A4, Hox-A5, Hox-A7, Hox-A9, Hox-A10, Hox-A11, Hox-A13, Hox-B1, Hox-B2, Hox-B3, Hox-B4, Hox-B5, Hox-B6, Hox-B7, Hox-B8, Hox-B9, Hox-B13, Hox-C4, Hox-C5, Hox-C6, Hox-C8, Hox-C9, Hox-C10, Hox-C11, Hox-C12, Hox-C13, Hox-D1, Hox-D3, Hox-D4, Hox-D8, Hox-D9, Hox-D10, Hox-D11, Hox-D12, Hox-D13, HOPX, IRX, IRX1, IRX2, IRX3, IRX4, IRX5, IRX6, MKX, LMX, LMX1A, LMX1B, MEIS, MEIS1, MEIS2, MEOX2, MNX1, MSX, MSX1, MSX2, NANOG, NKX, NKX2-1, NKX2-2, NKX2-3, NKX2-5, NKX3-1, NKX3-2, NKX6-1, NKX6-2, NOBOX, PBX, PBX1, PBX2, PBX3, PHF, PHF1, PHF3, PHF6, PHF8, PHF10, PHF16, PHF17, PHF20, PHF21A, PHOX, PHOX2A, PHOX2B, PITX, PITX1, PITX2, PITX3, a POU domain transcription factor, PIT-1, BRN-3, BRN-3A, BRN-3B, BRN-3C, an octamer transcription factor, OTX, OTX1, OTX2, PDX1, SATB2, SHOX2, SIX, SIX1, SIX2, SIX3, SIX4, SIX5, VAX1, ZEB, ZEB1, or ZEB2), a paired box transcription factor (such as, e.g., PAX, PAX1, PAX2, PAX3, PAX4, PAX5, PAX6, PAX7, PAX8, PAX9, PRRX, PRRX1, PRRX2, or RAX), a fork head/winged helix transcription factor (such as, e.g., E2F, E2F1, E2F2, E2F3, E2F4, E2F5, a FOX protein, FOXA1, FOXA2m, FOXA3, FOXC1, FOXC2, FOXD3, FOXD4, FOXE1, FOXE3, FOXF1, FOXG1, FOXH1, FOXI1, FOXJ1, FOXJ2, FOXK1, FOXK2, FOXL2, FOXM1, FOXN1, FOXN3, FOXO1, FOXO3, FOXO4, FOXP1, FOXP2, FOXP3, or FOXP4), a heat shock factor (such as, e.g., HSF1, HSF2, or HSF4), a tryptophan cluster transcription factor (such as, e.g., ELF, ELF2, ELF4, ELF5, EGF, ELK, ELK1, ELK3, ELK4, ERF, ETS, ETS1, ETS2, ERG, SPIB, ETV, ETV1, ETV4, ETV5, ETV6, FLI1, an interferon regulatory factors, IRF1, IRF2, IRF3, IRF4, IRF5, IRF6, IRF7, IRF8, MYB, or MYBL2), or a TEA domain transcription factor (such as, e.g., TEAD1, TEAD2, TEAD3, or TEAD4); a β-scaffold transcription factor with minor groove contact, particularly a Rel homology region (RHR) transcription factor (such as, e.g., NF-κB, NF-κB1, NF-κB2, REL, RELA, RELB, NFAT, NFATC1, NFATC2, NFATC3, NFATC4, or NFAT5), a STAT protein (such as, e.g., STAT1, STAT2, STAT3, STAT4, STAT5, or STAT6), a p53 transcription factor (such as, e.g., p53, a T-box protein, TBX1, TBX2, TBX3, TBX5, TBX19, TBX21, TBX22, TBR1, TBR2, TFT, MYRF, or TP63), a MADS box transcription factor (such as, e.g., Mef2, Mef2A, Mef2B, Mef2C, Mef2D, or SRF), a TATA-binding protein (such as, e.g., TBP or TBPL1), a high-mobility group (HMG) transcription factor (such as, e.g., BBX, HMGB, HMGB1, HMGB2, HMGB3, HMGB4, HMGN, HMGN1, HMGN2, HMGN3, HMGN4, HNF, HNF1A, HNF1B, LEF1, SOX, SOX1, SOX2, SOX3, SOX4, SOX5, SOX6, SOX8, SOX9, SOX10, SOX11, SOX12, SOX13, SOX14, SOX15, SOX18, SOX21, SRY, SSRP1, TCF, TCF3, TCF4, TOX, TOX1, TOX2, TOX3, or TOX4), a grainyhead transcription factor (such as, e.g., TFCP2), a cold-shock domain transcription factor (such as, e.g., CSDA or YBX1), or a Runt transcription factor (such as, e.g., CBF, CBFA2T2, CBFA2T3, RUNX1, RUNX2, RUNX3, or RUNX1T1); or any other transcription factor, particularly a copper fist protein, an HMGI(Y) protein (such as, e.g., HMGA, HMGA1, HMGA2, or HBP1), a pocket domain transcription factor (such as, e.g., Rb, RBL1, or RBL2), an AP2/EREBP-related factor (such as, e.g., Apetala 2, EREBP, or B3), ARID, ARID1A, ARID1B, ARID2, ARID3A, ARID3B, ARID4A, CAP, IFI, IFI16, IFI35, MLL, MLL2, MLL3, T1, MNDA, NFY, NFYA, NFYB, or NFYC.

The peptide/protein which can be used as cargo for the conjugate may also be a therapeutic peptide or protein, such as, for example, insulin, GLP-1, GLP-2, semaglutide, liraglutide, exenatide, exendin-4, lixisenatide, taspoglutide, albiglutide, dulaglutide, langlenatide, beinaglutide, efpeglenatide, oxyntomodulin, teduglutide, elsiglutide, elamipretide, recombinant factor Vila, eptacog alfa, amylin, pramlintide, octreotide, lanreotide, pasireotide, goserelin, buserelin, leptin, metreleptin, peptide YY, glatiramer, leuprolide, desmopressin, osteocalcin, human growth hormone, somapacitan, fibroblast growth factor 21, a glycopeptide antibiotic, vancomycin, teicoplanin, telavancin, bleomycin, ramoplanin, decaplanin, bortezomib, cosyntropin, chorionic gonadotropin, menotropin, sermorelin, gonadotropin-releasing hormone, somatropin, calcitonin, pentagastrin, oxytocin, neseritide, anakinra, enfuvirtide, pegvisomant, dornase alfa, lepirudin, anidulafungin, eptifibatide, interferon alfacon-1, interferon alpha-2a, interferon alpha-2b, interferon beta-1a, interferon beta-1b, interferon gamma-1b, peginterferon alfa-2a, peginterferon alfa-2b, peginterferon beta-1a, fibrinolysin, vasopressin, aldesleukin, an epoetin, epoetin alfa, darbepoetin alfa, epoetin beta, epoetin delta, epoetin omega, epoetin zeta, epoetin theta, albupoetin, filgrastim, PEG-filgrastim, interleukin-11, cyclosporine, glucagon, urokinase, viomycin, thyrotropin-releasing hormone, leucine-enkephalin, methionine-enkephalin, substance P, adrenocorticotropic hormone, parathyroid hormone, teriparatide, parathyroid hormone-related protein, abaloparatide, linaclotide, carfilzomib, icatibant, ecallantide, cilengitide, abciximab, ranibizumab, alefacept, romiplostim, anakinra, abatacept, or belatacept.

The peptide/protein which can be used as cargo for the conjugate may also be an enzyme, such as, e.g., a nuclease, a protease, a DNase, dornase α, an RNase, onconase, ranpirnase, bovine seminal RNase, RNase T1, α-sarcin, RNase P, actibind, RNase T2, alkaline phosphatase, asfotase alfa, aspartylglucosaminidase, aspartoacylase, α-mannosidase, lecithin cholesterol acyl transferase, thymidine phosphorylase, arylsulfatase A, cyclin-dependent kinase-like 5 protein, gliadin peptidase, aldehyde dehydrogenase, glutaminase, arginase, arginine deiminase, reteplase, streptokinase, urokinase, urate oxidase, adenosine deaminase, purine nucleoside phosphorylase, phenylalanine hydroxylase, phenylalanine ammonia lyase, superoxide dismutase, catalase, phosphotriesterase, organophosphorus anhydrolase, alcohol dehydrogenase, alcohol oxidase, a fibrinogenolytic enzyme, ancrod, batroxobin, cystathionine-β-synthase, paraoxonase 1, bleomycin hydrolase, human biphenyl hydrolase-like protein, methioninase, hyaluronidase, α-glucosidase, β-glucuronidase, β-galactosidase, α-galactosidase A, glucocerebrosidase, imiglucerase, ananain, comosain, ocriplasmin, acetylcholinesterase, butyrylcholinesterase, cocaine esterase, chondroitinase, collagenase, N-acetylgalactosamine-4-sulfatase, iduronate-2-sulfatase, α-L-iduronidase, porphobilinogen, oxalate decarboxylase, N-sulfoglucosamine sulfohydrolase, acetyl CoA α-glucosaminide acetyltransferase, N-acetylglucosamine-6-sulfatase, N-α-acetylglucosaminidase, N-acetylgalactosamine-6-sulfate sulfatase, tripeptidyl peptidase 1, or phosphoglycerate kinase.

The peptide/protein which can be used as cargo may also be an antibody, particularly a monoclonal antibody, such as, e.g., 3F8, 8H9, abagovomab, abciximab, abituzumab, abrezekimab, abrilumab, actoxumab, adalimumab, adecatumumab, atidortoxumab, aducanumab, afasevikumab, afelimomab, afutuzumab, alacizumab pegol, alemtuzumab, alirocumab, altumomab pentetate, amatuximab, anatumomab mafenatox, andecaliximab, anetumab ravtansine, anifrolumab, anrukinzumab, apolizumab, aprutumab ixadotin, arcitumomab, ascrinvacumab, aselizumab, atezolizumab, atinumab, atorolimumab, avelumab, azintuxizumab vedotin, bapineuzumab, basiliximab, bavituximab, BCD-100, bectumomab, begelomab, belantamab mafodotin, belimumab, bemarituzumab, benralizumab, berlimatoxumab, bersanlimab, bertilimumab, besilesomab, bevacizumab, bezlotoxumab, biciromab, bimagrumab, bimekizumab, birtamimab, bivatuzumab mertansine, BIVV009, bleselumab, blinatumomab, blontuvetmab, blosozumab, bococizumab, brazikumab, brentuximab vedotin, briakinumab, brodalumab, brolucizumab, brontictuzumab, burosumab, cabiralizumab, camidanlumab tesirine, camrelizumab, canakinumab, cantuzumab mertansine, cantuzumab ravtansine, caplacizumab, capromab pendetide, carlumab, carotuximab, catumaxomab, cbr96-doxorubicin immunoconjugate, cedelizumab, cemiplimab, cergutuzumab amunaleukin, certolizumab pegol, cetrelimab, cetuximab, cibisatamab, citatuzumab bogatox, cixutumumab, clazakizumab, clenoliximab, clivatuzumab tetraxetan, codrituzumab, cofetuzumab pelidotin, coltuximab ravtansine, conatumumab, concizumab, cosfroviximab, crenezumab, crizanlizumab, crotedumab, CR6261, cusatuzumab, dacetuzumab, daclizumab, dalotuzumab, dapirolizumab pegol, daratumumab, dectrekumab, demcizumab, denintuzumab mafodotin, denosumab, depatuxizumab mafodotin, derlotuximab biotin, detumomab, dezamizumab, dinutuximab, diridavumab, domagrozumab, dorlimomab aritox, drozitumab, DS-8201, duligotuzumab, dupilumab, durvalumab, dusigitumab, duvortuxizumab, ecromeximab, eculizumab, edobacomab, edrecolomab, efalizumab, efungumab, eldelumab, elezanumab, elgemtumab, elotuzumab, elsilimomab, emactuzumab, emapalumab, emibetuzumab, emicizumab, enapotamab edotin, enavatuzumab, enfortumab vedotin, enlimomab pegol, enoblituzumab, enokizumab, enoticumab, ensituximab, epitumomab cituxetan, epratuzumab, eptinezumab, erenumab, erlizumab, ertumaxomab, etaracizumab, etigilimab, etrolizumab, evinacumab, evolocumab, exbivirumab, fanolesomab, faralimomab, faricimab, farletuzumab, fasinumab, FBTA05, felvizumab, fezakinumab, fibatuzumab, ficlatuzumab, figitumumab, firivumab, flanvotumab, fletikumab, flotetuzumab, fontolizumab, foralumab, foravirumab, fremanezumab, fresolimumab, frunevetmab, fulranumab, futuximab, galcanezumab, galiximab, gancotamab, ganitumab, gantenerumab, gatipotuzumab, gavilimomab, gedivumab, gemtuzumab ozogamicin, gevokizumab, gilvetmab, gimsilumab, girentuximab, glembatumumab vedotin, golimumab, gomiliximab, gosuranemab, guselkumab, ianalumab, ibalizumab, IBI308, ibritumomab tiuxetan, icrucumab, idarucizumab, ifabotuzumab, igovomab, iladatuzumab vedotin, IMAB362, imalumab, imaprelimab, imciromab, imgatuzumab, inclacumab, indatuximab ravtansine, indusatumab vedotin, inebilizumab, infliximab, intetumumab, inolimomab, inotuzumab ozogamicin, ipilimumab, iomab-b, iratumumab, isatuximab, iscalimab, istiratumab, itolizumab, ixekizumab, keliximab, labetuzumab, lacnotuzumab, ladiratuzumab vedotin, lampalizumab, lanadelumab, landogrozumab, laprituximab emtansine, larcaviximab, lebrikizumab, lemalesomab, lendalizumab, lenvervimab, lenzilumab, lerdelimumab, leronlimab, lesofavumab, letolizumab, lexatumumab, libivirumab, lifastuzumab vedotin, ligelizumab, loncastuximab tesirine, losatuxizumab vedotin, lilotomab satetraxetan, lintuzumab, lirilumab, lodelcizumab, lokivetmab, lorvotuzumab mertansine, lucatumumab, lulizumab pegol, lumiliximab, lumretuzumab, lupartumab amadotin, lutikizumab, MABp1, mapatumumab, margetuximab, marstacimab, maslimomab, mavrilimumab, matuzumab, mepolizumab, metelimumab, milatuzumab, minretumomab, mirikizumab, mirvetuximab soravtansine, mitumomab, modotuximab, mogamulizumab, monalizumab, morolimumab, mosunetuzumab, motavizumab, moxetumomab pasudotox, muromonab-CD3, nacolomab tafenatox, namilumab, naptumomab estafenatox, naratuximab emtansine, narnatumab, natalizumab, navicixizumab, navivumab, naxitamab, nebacumab, necitumumab, nemolizumab, NEOD001, nerelimomab, nesvacumab, netakimab, nimotuzumab, nirsevimab, nivolumab, nofetumomab merpentan, obiltoxaximab, obinutuzumab, ocaratuzumab, ocrelizumab, odulimomab, ofatumumab, olaratumab, oleclumab, olendalizumab, olokizumab, omalizumab, OMS721, onartuzumab, ontuxizumab, onvatilimab, opicinumab, oportuzumab monatox, oregovomab, orticumab, otelixizumab, otilimab, otlertuzumab, oxelumab, ozanezumab, ozoralizumab, pagibaximab, palivizumab, pamrevlumab, panitumumab, pankomab, panobacumab, parsatuzumab, pascolizumab, pasotuxizumab, pateclizumab, patritumab, PDR001, pembrolizumab, pemtumomab, perakizumab, pertuzumab, pexelizumab, pidilizumab, pinatuzumab vedotin, pintumomab, placulumab, plozalizumab, pogalizumab, polatuzumab vedotin, ponezumab, porgaviximab, prasinezumab, prezalizumab, priliximab, pritoxaximab, pritumumab, PRO 140, quilizumab, racotumomab, radretumab, rafivirumab, ralpancizumab, ramucirumab, ranevetmab, anibizumab, raxibacumab, ravagalimab, ravulizumab, refanezumab, regavirumab, remtolumab, reslizumab, rilotumumab, rinucumab, risankizumab, rituximab, rivabazumab pegol, robatumumab, rmab, roledumab, romilkimab, romosozumab, rontalizumab, rosmantuzumab, rovalpituzumab tesirine, rovelizumab, rozanolixizumab, ruplizumab, SA237, sacituzumab govitecan, samalizumab, samrotamab vedotin, sapelizumab, sarilumab, satralizumab, satumomab pendetide, secukinumab, selicrelumab, seribantumab, setoxaximab, setrusumab, sevirumab, sibrotuzumab, SGN-CD19A, SHP647, sifalimumab, siltuximab, simtuzumab, siplizumab, sirtratumab vedotin, sirukumab, sofituzumab vedotin, solanezumab, solitomab, sonepcizumab, sontuzumab, spartalizumab, stamulumab, sulesomab, suptavumab, sutimlimab, suvizumab, suvratoxumab, tabalumab, tacatuzumab etraxetan, tadocizumab, talacotuzumab, talizumab, tamtuvetmab, tanezumab, taplitumomab paptox, tarextumab, tavolimab, tefibazumab, telimomab aritox, telisotuzumab vedotin, tenatumomab, teneliximab, teplizumab, tepoditamab, teprotumumab, tesidolumab, tetulomab, tezepelumab, TGN1412, tibulizumab, tildrakizumab, tigatuzumab, timigutuzumab, timolumab, tiragotumab, tislelizumab, tisotumab vedotin, TNX-650, tocilizumab, tomuzotuximab, toralizumab, tosatoxumab, tositumomab, tovetumab, tralokinumab, trastuzumab, trastuzumab emtansine, TRBS07, tregalizumab, tremelimumab, trevogrumab, tucotuzumab celmoleukin, tuvirumab, ublituximab, ulocuplumab, urelumab, urtoxazumab, ustekinumab, utomilumab, vadastuximab talirine, vanalimab, vandortuzumab vedotin, vantictumab, vanucizumab, vapaliximab, varisacumab, varlilumab, vatelizumab, vedolizumab, veltuzumab, vepalimomab, vesencumab, visilizumab, vobarilizumab, volociximab, vonlerolizumab, vopratelimab, vorsetuzumab mafodotin, votumumab, vunakizumab, xentuzumab, XMAB-5574, zalutumumab, zanolimumab, zatuximab, zenocutuzumab, ziralimumab, zolbetuximab, or zolimomab aritox.

The nucleic acid which can be used as cargo for the conjugate according to the invention may be, e.g., a DNA, an RNA, an oligonucleotide, a deoxyoligonucleotide, a peptide nucleic acid (PNA), a locked nucleic acid, an aptamer, an aptazyme, a ribozyme, a DNAzyme, an antisense DNA, an antisense RNA, an siRNA, an siDNA, or a miRNA.

Moreover, the cargo may be a gene editing system or a component thereof. In particular, the cargo may be a CRISPR-Cas gene editing component, such as, e.g., a Cas9 protein, a Cas9-encoding nucleic acid, a guide RNA (particularly a single guide RNA), a truncated guide RNA (see, e.g., Fu Y et al., Nat Biotechnol, 2014, 32(3): 279-284), or a donor nucleic acid (e.g., a donor RNA or donor DNA, which may be single-stranded or double-stranded, and may further be linear or circular; see, e.g., US 2010/0047805, US 2011/0207221, or Richardson CD et al., Nat Biotechnol, 2016, 34(3): 339-44). The various components required for a gene editing system to be effective can be delivered into a cell using, e.g., several different types of silica-encapsulated polyphenol-peptide conjugates according to the invention, wherein each conjugate contains only one of the gene editing components as cargo; alternatively, a single silica-encapsulated polyphenol-peptide conjugate may also contain two or more different components of the gene editing system as cargos, all of which are attached to the peptide in the conjugate. The cargo may also be any one of the CRISPR-Cas components (or donor nucleic acids) referred to in: Sander JD et al., Nat Biotechnol, 2014, 32(4): 347-55; Fu Y et al., Nat Biotechnol, 2014, 32(3): 279-284; Oude Blenke E et al., J Control Release, 2016, 244(Pt B): 139-48; Richardson CD et al., Nat Biotechnol, 2016, 34(3): 339-44; Liu C et al., J Control Release, 2017, 266: 17-26; Glass Z et al., Trends Biotechnol, 2018, 36(2): 173-85; Lino CA et al., Drug Deliv, 2018, 25(1): 1234-57; WO 2014/186585; or in any reference cited in any of the aforementioned documents.

The active pharmaceutical ingredient (or therapeutic agent) which can be used as cargo for the conjugate according to the invention may be, e.g., a small molecule therapeutic agent. In particular, it may be, e.g., a therapeutic agent that binds to a transcription factor, a DNA-binding inhibitor of a transcription factor, a bromodomain or BET inhibitor (e.g., I-BET 762 (or GSK525762), I-BET 151 (or GSK1210151A), OTX-015, TEN-010, CPI-203, CPI-0610, olinone, LY294002, or JQ1), or a topoisomerase inhibitor (e.g., irinotecan, topotecan, belotecan, rubitecan, cositecan, gimatecan, exatecan, lurtotecan, silatecan, camptothecin, lamellarin D, amsacrine, etoposide, teniposide, doxorubicin, aclarubicin, daunorubicin, epirubicin, idarubicin, amrubicin, pirarubicin, valrubicin, zorubicin, mitoxantrone, or pixantrone).

The cargo(s) contained in the polyphenol-peptide conjugate may also comprise a cleavable linker. Accordingly, the cargo be, e.g., a peptide or protein, a nucleic acid, a lipid, a sugar, or an active pharmaceutical ingredient, wherein said peptide or protein, said nucleic acid, said lipid, said sugar, or said active pharmaceutical ingredient is substituted with (or modified to contain) a cleavable linker, and wherein the cargo can be covalently bound via the cleavable linker to the peptide (which is comprised in the polyphenol-peptide conjugate). The cleavable linker may be, e.g., a chemically cleavable linker, an enzymatically cleavable linker, a pH-sensitive linker, an acid-labile linker, or a disulfide linker. Exemplary cleavable linkers include any of those described in: Leriche G et al., Bioorg Med Chem, 2012, 20(2): 571-82; Rudolf GC et al., Curr Opin Chem Biol, 2013, 17(1): 110-7; Yang Y et al., Mol Cell Proteomics, 2013, 12(1): 237-44; Nolting B et al., Methods Mol Biol, 2013, 1045: 71-100; Jain N et al., Pharm Res, 2015, 32: 3526-40; Chang M et al., J Drug Target, 2016, 24(6): 475-91; Saneyoshi H et al., J Org Chem, 2017, 82(3): 1796-802; or Yang Y et al., Methods Mol Biol, 2017, 1491: 185-203.

The polyphenol-peptide conjugate, however, does not necessarily have to contain any cargo. For example, the conjugate according to the invention without cargo can also be used for the intracellular delivery of the polyphenol, e.g., for therapeutic uses. Polyphenols (including, e.g., quercetin) are known to exert beneficial physiological activities, including antioxidative effects, and have been proposed as therapeutic agents (Ullah MF et al., Asian Pac J Cancer Prev, 2008, 9(2): 187-95; Ramos S, Mol Nutr Food Res, 2008, 52(5): 507-26; Darvesh AS et al., Expert Rev Neurother, 2010, 10(5): 729-45; Scapagnini G et al., Adv Exp Med Biol, 2010, 698: 27-35; Choi DY et al., Brain Res Bull, 2012, 87(2-3): 144-53; Daglia M, Curr Opin Biotechnol, 2012, 23(2): 174-81; Notas G et al., J Nutr Biochem, 2012, 23(6): 656-66; Khurana S et al., Can J Physiol Pharmacol, 2013, 91(3): 198-212; Bhullar KS et al., Oxid Med Cell Longev, 2013, 891748; Habtemariam S et al., Curr Pharm Biotechnol, 2014, 15(4):391-400; Shay J et al., Oxid Med Cell Longev, 2015, 181260; Lakey-Beitia J et al., Mol Neurobiol, 2015, 51(2): 466-79). Moreover, the intracellular delivery of a polyphenol, using the silica-encapsulated polyphenol-peptide conjugate according to the present invention, can also be employed as a protective strategy for cells under pharmaceutical stress, e.g., during chemotherapy.

### Silica encapsulation of the polyphenol-peptide conjugate

The polyphenol-peptide conjugate according to the present invention is preferably encapsulated by a silica shell. A silica-encapsulated polyphenol-peptide conjugate can be prepared by subjecting a non-encapsulated polyphenol-peptide conjugate to silica precipitation in aqueous solution.

Accordingly, the silica-encapsulated polyphenol-peptide conjugate can be prepared by subjecting an aqueous solution of the polyphenol-peptide conjugate to silicic acid and thereby induce silica precipitation to obtain silica particles containing the polyphenol-peptide conjugate encapsulated by a silica shell. This allows to obtain silica particles comprising the polyphenol-peptide conjugate, wherein said conjugate is encapsulated by a silica shell, and wherein the particles are preferably spherical and homogenous. Silica precipitation is preferably induced by adding freshly generated silicic acid to the aqueous solution of the polyphenol-peptide conjugate. The aqueous solution of the polyphenol-peptide conjugate may be, e.g., an aqueous phosphate buffer solution (about pH 7). The silicic acid can be generated, e.g., from tetramethoxysilane and aqueous hydrochloric acid. In particular, the silica encapsulation can be conducted in accordance with or in analogy to the procedure described in Example 1.

### Pharmaceutical formulations

The silica particles or encapsulated conjugates according to the invention may be administered as such, or they may be formulated as medicaments or pharmaceutical compositions. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The silica particles or conjugates or the above described pharmaceutical compositions comprising them may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, or vaginal administration.

If said particles, conjugates or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the particles, conjugates or pharmaceutical compositions, and/or by using infusion techniques. For parenteral administration, the particles or conjugates are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said particles, conjugates or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the particles or conjugates may be combined with various sweetening or flavoring agents, coloring matter or dyes, with emulsifying and/or suspending agents and with diluents such as water, ethanol, propylene glycol and glycerin, and combinations thereof.

Alternatively, said particles, conjugates or pharmaceutical compositions can be administered in the form of a suppository or pessary, or may be applied topically in the form of a gel, hydrogel, lotion, solution, cream, ointment or dusting powder. The particles or conjugates may also be dermally or transdermally administered, for example, by the use of a skin patch.

Said particles, conjugates or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides, copolymers of L-glutamic acid and gamma-ethyl-L-glutamate, poly(2-hydroxyethyl methacrylate), ethylene vinyl acetate, or poly-D-(-)-3-hydroxybutyric acid. Sustained-release pharmaceutical compositions also include liposomally entrapped particles or conjugates. The present invention thus also relates to liposomes containing the particles or conjugates of the invention.

Said particles, conjugates or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the particles or conjugates for pulmonary administration, particularly inhalation. Such dry powders may be prepared by spray drying under conditions which result in a substantially amorphous glassy or a substantially crystalline bioactive powder. Accordingly, dry powders of the particles or conjugates of the present invention can be made according to an emulsification/spray drying process.

For topical application to the skin, said particles, conjugates or pharmaceutical compositions can be formulated as a suitable ointment containing the active ingredient suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the particles, conjugates or the pharmaceutical compositions provided herein, wherein the corresponding particles, conjugates or pharmaceutical composition is/are to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. Preferred routes of administration are oral administration or parenteral administration.

Typically, a physician will determine the actual dosage which will be most suitable for an individual subject. The specific dose level and frequency of dosage for any particular individual subject may be varied and will depend upon a variety of factors including the activity of the specific active ingredient employed, the metabolic stability and length of action of that ingredient, the age, body weight, general health, sex, diet, mode and time of administration, rate of excretion, drug combination, the severity of the particular condition, and the individual subject undergoing therapy.

The subject or patient to be treated in accordance with the present invention may be an animal (e.g., a non-human animal). Preferably, the subject/patient is a mammal. More preferably, the subject/patient is a human (e.g., a male human or a female human) or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient to be treated in accordance with the invention is a human.

### Definitions

The following definitions apply throughout the present specification and the claims, unless specifically indicated otherwise.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₅ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₅ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₅ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₅ alkylene" denotes an alkylene group having 1 to 5 carbon atoms, and the term "C₀₋₅ alkylene" indicates that a covalent bond (corresponding to the option "C₀ alkylene") or a C₁₋₅ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). If the aryl is a bridged and/or fused ring system which contains, besides one or more aromatic rings, at least one non-aromatic ring (e.g., a saturated ring or an unsaturated alicyclic ring), then one or more carbon ring atoms in each non-aromatic ring may optionally be oxidized (i.e., to form an oxo group). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "arene" refers to an aromatic hydrocarbon (i.e., a compound consisting of carbon atoms and hydrogen atoms), which may be a monocyclic aromatic ring or a bridged and/or fused ring system containing at least one aromatic ring (e.g., a ring system composed of two, three or four fused rings, wherein at least one of these fused rings is aromatic; or a bridged ring system composed of two, three or four rings, wherein at least one of these bridged rings is aromatic). If the arene is a bridged and/or fused ring system which contains, besides one or more aromatic rings, at least one non-aromatic ring (e.g., a saturated ring or an unsaturated alicyclic ring), then one or more carbon ring atoms in each non-aromatic ring may optionally be oxidized (i.e., to form an oxo group). "Arene" may, e.g., refer to benzene, naphthalene, dialin (i.e., 1,2-dihydronaphthalene), tetralin (i.e., 1,2,3,4-tetrahydronaphthalene), indane, indene (e.g., 1H-indene), anthracene, phenanthrene, 9H-fluorene, or azulene. Unless defined otherwise, an "arene" preferably has 6 to 14 ring atoms, more preferably 6 to 10 ring atoms.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, particularly preferred examples of a "heteroaryl" include pyridinyl (e.g., 2-pyridyl, 3-pyridyl, or 4-pyridyl), imidazolyl, thiazolyl, 1H-tetrazolyl, 2H-tetrazolyl, thienyl (i.e., thiophenyl), or pyrimidinyl.

As used herein, the term "heteroarene" refers to an aromatic compound, which may be a monocyclic aromatic ring or a bridged ring and/or fused ring system containing at least one aromatic ring (e.g., a ring system composed of two or three fused rings, wherein at least one of these fused rings is aromatic; or a bridged ring system composed of two or three rings, wherein at least one of these bridged rings is aromatic), wherein said monocyclic aromatic ring or said bridged ring and/or fused ring system comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic compound may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroarene" may, e.g., refer to thiophene (i.e., thiofuran), benzo[b]thiophene, naphtho[2,3-b]thiophene, thianthrene, furan, benzofuran, isobenzofuran, chromane, chromene (e.g., 2H-1-benzopyran or 4H-1-benzopyran), isochromene (e.g., 1H-2-benzopyran), chromone (i.e., chromen-4-one), xanthene, phenoxathiin, pyrrole (e.g., 1H-pyrrole), imidazole, pyrazole, pyridine, pyrazine, pyrimidine, pyridazine, indole (e.g., 3H-indole), isoindole, indazole, indolizine, purine, quinoline, isoquinoline, phthalazine, naphthyridine, quinoxaline, cinnoline, pteridine, carbazole, β-carboline, phenanthridine, acridine, perimidine, phenanthroline (e.g., [1,10]phenanthroline, [1,7]phenanthroline, or [4,7]phenanthroline), phenazine, thiazole, isothiazole, phenothiazine, oxazole, isoxazole, oxadiazole (e.g., 1,2,4-oxadiazole, 1,2,5-oxadiazole (i.e., furazan), or 1,3,4-oxadiazole), thiadiazole (e.g., 1,2,4-thiadiazole, 1,2,5-thiadiazole, or 1,3,4-thiadiazole), phenoxazine, pyrazolo[1,5-a]pyrimidine, 1,2-benzoisoxazole, benzothiazole, benzothiadiazole, benzoxazole, benzisoxazole, benzimidazole, benzo[b]thiophene, triazole (e.g., 1H-1,2,3-triazole, 2H-1,2,3-triazole, 1H-1,2,4-triazole, or 4H-1,2,4-triazole), benzotriazole, 1H-tetrazole, 2H-tetrazole, triazine (e.g., 1,2,3-triazine, 1,2,4-triazine, or 1,3,5-triazine), furo[2,3-c]pyridine, dihydrofuropyridine (e.g., 2,3-dihydrofuro[2,3-c]pyridine or 1,3-dihydrofuro[3,4-c]pyridine), imidazopyridine (e.g., imidazo[1,2-a]pyridine or imidazo[3,2-a]pyridine), quinazoline, thienopyridine, tetrahydrothienopyridine (e.g., 4,5,6,7-tetrahydrothieno[3,2-c]pyridine), dibenzofuran, 1,3-benzodioxole, benzodioxan (e.g., 1,3-benzodioxan or 1,4-benzodioxan), or coumarin. Unless defined otherwise, the term "heteroarene" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members. Moreover, unless defined otherwise, particularly preferred examples of a "cycloalkyl" include cyclohexyl or cyclopropyl, particularly cyclohexyl.

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized. Moreover, unless defined otherwise, particularly preferred examples of a "heterocycloalkyl" include tetrahydropyranyl, piperidinyl, piperazinyl, morpholinyl, pyrrolidinyl, or tetrahydrofuranyl.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "halogen" refers to fluoro (-F), chloro (-Cl), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

As used herein, the term "small molecule" refers to any molecule, particularly any organic molecule (i.e., any molecule containing, *inter alia,* carbon atoms), that has a molecular weight of equal to or less than about 900 Da, preferably of equal to or less than about 500 Da. The molecular weight of a molecule can be determined using methods known in the art, such as, e.g., mass spectrometry (e.g., electrospray ionization mass spectrometry (ESI-MS) or matrix-assisted laser desorption/ionization mass spectrometry (MALDI-MS)), gel electrophoresis (e.g., polyacrylamide gel electrophoresis using sodium dodecyl sulfate (SDS-PAGE)), hydrodynamic methods (e.g., gel filtration chromatography or gradient sedimentation), or static light scattering (e.g., multi-angle light scattering (MALS)), and is preferably determined using mass spectrometry.

As used herein, the term "polyphenol" refers to a compound comprising (or consisting of) a monocyclic aromatic ring or a polycyclic (e.g., bicyclic, tricyclic or tetracyclic) aromatic ring system, wherein said ring or said ring system is substituted with two or more hydroxy (-OH) groups. The polycyclic aromatic ring system may be, e.g., a bridged ring, fused ring and/or spiro ring system, wherein at least one ring comprised in said ring system is aromatic.

As used herein, the term "amino acid" refers, in particular, to any one of the 20 standard proteinogenic α-amino acids (i.e., Ala, Arg, Asn, Asp, Cys, Glu, Gin, Gly, His, Ile, Leu, Lys, Met, Phe, Pro, Ser, Thr, Trp, Tyr, or Val) but also to a non-proteinogenic and/or non-standard α-amino acid (such as, e.g., ornithine, citrulline, homolysine, pyrrolysine, 4-hydroxyproline, α-methylalanine (i.e., 2-aminoisobutyric acid), norvaline, norleucine, terleucine (tert-leucine), labionin, or an alanine or glycine that is substituted at the side chain with a carbocyclyl or heterocyclyl group (e.g., with a cycloalkyl, a heterocycloalkyl, an aryl, or a heteroaryl) like, e.g., cyclopentylalanine, cyclohexylalanine, phenylalanine, naphthylalanine, pyridylalanine, thienylalanine, cyclohexylglycine, or phenylglycine), or a β-amino acid (e.g., β-alanine), a γ-amino acid (e.g., γ-aminobutyric acid, isoglutamine, or statine) or a δ-amino acid, or any other compound comprising at least one carboxylic acid group and at least one amino group. Unless defined otherwise, the term "amino acid" preferably refers to an α-amino acid, more preferably to any one of the 20 standard proteinogenic α-amino acids (which may be in the form of the L-isomer or the D-isomer but are preferably in the form of the L-isomer).

The terms "peptide" and "protein" are used herein interchangeably and refer to a polymer of two or more amino acids linked via amide bonds that are formed between an amino group of one amino acid and a carboxyl group of another amino acid. The amino acids comprised in the peptide or protein, which are also referred to as amino acid residues, may be selected from the 20 standard proteinogenic α-amino acids (i.e., Ala, Arg, Asn, Asp, Cys, Glu, Gin, Gly, His, Ile, Leu, Lys, Met, Phe, Pro, Ser, Thr, Trp, Tyr, and Val) but also from non-proteinogenic and/or non-standard α-amino acids (such as, e.g., ornithine, citrulline, homolysine, pyrrolysine, 4-hydroxyproline, α-methylalanine (i.e., 2-aminoisobutyric acid), norvaline, norleucine, terleucine (tert-leucine), formylglycine (i.e., C_{α}-formylglycine), labionin, or an alanine or glycine that is substituted at the side chain with a carbocyclyl or heterocyclyl group (e.g., with a cycloalkyl, a heterocycloalkyl, an aryl, or a heteroaryl) like, e.g., cyclopentylalanine, cyclohexylalanine, phenylalanine, naphthylalanine, pyridylalanine, thienylalanine, cyclohexylglycine, or phenylglycine) as well as β-amino acids (e.g., β-alanine), γ-amino acids (e.g., γ-aminobutyric acid, isoglutamine, or statine) and δ-amino acids. Preferably, the amino acid residues comprised in the peptide or protein are selected from α-amino acids, more preferably from the 20 standard proteinogenic α-amino acids (which can be present as the L-isomer or the D-isomer, and are preferably all present as the L-isomer). The peptide or protein may be unmodified or may be modified, e.g., at its N-terminus, at its C-terminus and/or at a functional group in the side chain of any of its amino acid residues (particularly at the side chain functional group of one or more Lys, His, Ser, Thr, Tyr, Cys, Asp, Glu, and/or Arg residues). Such modifications may include, e.g., the attachment of any of the protecting groups described for the corresponding functional groups in: Wuts PGM, Greene's protective groups in organic synthesis, 5th edition, John Wiley & Sons, 2014. Such modifications may also include the covalent attachment of one or more polyethylene glycol (PEG) chains (forming a PEGylated peptide or protein), the glycosylation and/or the acylation with one or more fatty acids (e.g., one or more C₈₋₃₀ alkanoic or alkenoic acids; forming a fatty acid acylated peptide or protein). Moreover, such modified peptides or proteins may also include peptidomimetics, provided that they contain at least two amino acids that are linked via an amide bond (formed between an amino group of one amino acid and a carboxyl group of another amino acid). The amino acid residues comprised in the peptide or protein may, e.g., be present as a linear molecular chain (forming a linear peptide or protein) or may form one or more rings (corresponding to a cyclic peptide or protein). The peptide or protein may also form oligomers consisting of two or more identical or different molecules.

The term *"in vitro"* is used herein in the sense of "outside a living human or animal body", which includes, in particular, experiments performed with cells, cellular or subcellular extracts, and/or biological molecules in an artificial environment such as an aqueous solution or a culture medium which may be provided, e.g., in a flask, a test tube, a Petri dish, a microtiter plate, etc.

As used herein, and unless contradicted by context, the term "treatment" or "treating" (of a disease or disorder) refers to curing, alleviating, reducing or preventing one or more symptoms or clinically relevant manifestations of a disease or disorder, or to alleviating, reversing or eliminating the disease or disorder, or to preventing the onset of the disease or disorder, or to preventing, reducing or delaying the progression of the disease or disorder. For example, the "treatment" of a subject or patient in whom no symptoms or clinically relevant manifestations of the respective disease or disorder have been identified is a preventive or prophylactic treatment, whereas the "treatment" of a subject or patient in whom symptoms or clinically relevant manifestations of the respective disease or disorder have been identified may be, e.g., a curative or palliative treatment. Each one of these forms of treatment may be considered as a distinct aspect of the present invention.

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, *inter alia,* comprise curative treatment (preferably leading to a complete response and eventually to healing of the disorder or disease), palliative treatment (including symptomatic relief), or prophylactic treatment (including prevention) of the disorder or disease.

As used herein, unless explicitly indicated otherwise or contradicted by context, the terms "a", "an" and "the" are used interchangeably with "one or more" and "at least one".

It is to be understood that wherever numerical ranges are provided/disclosed herein, all values and subranges encompassed by the respective numerical range are meant to be encompassed within the scope of the present invention. Accordingly, the present invention specifically and individually relates to each value that falls within a numerical range disclosed herein, as well as each subrange encompassed by a numerical range disclosed herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint. If the term "about" is used in connection with the endpoint of an open-ended range, it preferably refers to the corresponding range starting from the lower endpoint -10% or from the upper endpoint +10%, more preferably to the range starting from the lower endpoint -5% or from the upper endpoint +5%, and even more preferably to the open-ended range defined by the exact numerical value of the corresponding endpoint. If the term "about" is used in connection with a parameter that is quantified in integers, such as the number of amino acid residues in a given peptide or amino acid sequence, the numbers corresponding to ±10% or ±5% of the indicated numerical value are to be rounded to the nearest integer (using the tie-breaking rule "round half up").

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

It is to be understood that the present invention specifically relates to each and every combination of features and embodiments described herein, including any combination of general and/or preferred features/embodiments.

In this specification, a number of documents including patent applications and scientific literature are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

The reference in this specification to any prior publication (or information derived therefrom) is not and should not be taken as an acknowledgment or admission or any form of suggestion that the corresponding prior publication (or the information derived therefrom) forms part of the common general knowledge in the technical field to which the present specification relates.

The present invention particularly relates to the following items:
1. A polyphenol-peptide conjugate consisting of:
   - a peptide,
      wherein said peptide comprises the amino acid sequence Arg-Arg-X¹-Leu, wherein X¹ refers to one or two hydrophobic amino acids which are each independently selected from Ile, Leu, Val, Phe, Trp, and Met;
   - at least one polyphenol,
      wherein each polyphenol is covalently bound to the peptide;
   - and optionally at least one cargo,
      wherein each cargo, if present, is covalently bound to the peptide.
2. The polyphenol-peptide conjugate of item 1, wherein the peptide comprises the amino acid sequence Arg-Arg-X¹-Leu, wherein X¹ refers to one hydrophobic amino acid selected from Ile, Leu, and Val.
3. The polyphenol-peptide conjugate of item 1 or 2, wherein the peptide comprises the amino acid sequence Arg-Arg-Ile-Leu.
4. The polyphenol-peptide conjugate of any one of items 1 to 3, wherein the peptide consists of about 10 to about 40 amino acid residues, preferably of about 15 to about 30 amino acid residues, more preferably of about 20 amino acid residues.
5. The polyphenol-peptide conjugate of any one of items 1 to 4, wherein the peptide comprises or consists of the amino acid sequence CSSKKSGSYSGSKGSKRRIL.
6. The polyphenol-peptide conjugate of any one of items 1 to 5, wherein each of said at least one polyphenol is independently an arene or heteroarene, wherein said arene or said heteroarene is substituted with two or more -OH and is optionally further substituted with one or more R¹;
   wherein each R¹ is independently selected from C₁₋₅ alkyl, C₂₋₅ alkenyl, C₂₋₅ alkynyl, -(C₀₋₅ alkylene)-OH, -(C₀₋₅ alkylene)-O(C₁₋₅ alkyl), -(C₀₋₅ alkylene)-O(C₁₋₅ alkylene)-OH, -(C₀₋₅ alkylene)-O(C₁₋₅ alkylene)-O(C₁₋₅ alkyl), -(C₀₋₅ alkylene)-SH, -(C₀₋₅ alkylene)-S(C₁₋₅ alkyl), -(C₀₋₅ alkylene)-S(C₁₋₅ alkylene)-SH, -(C₀₋₅ alkylene)-S(C₁₋₅ alkylene)-S(C₁₋₅ alkyl), -(C₀₋₅ alkylene)-NH₂, -(C₀₋₅ alkylene)-NH(C₁₋₅ alkyl), -(C₀₋₅ alkylene)-N(C₁₋₅ alkyl)(C₁₋₅ alkyl), -(C₀₋₅ alkylene)-halogen, -(C₀₋₅ alkylene)-(C₁₋₅ haloalkyl), -(C₀₋₅ alkylene)-O-(C₁₋₅ haloalkyl), -(C₀₋₅ alkylene)-CN, -(C₀₋₅ alkylene)-NO₂, -(C₀₋₅ alkylene)-CHO, -(C₀₋₅ alkylene)-CO-(C₁₋₅ alkyl), -(C₀₋₅ alkylene)-COOH, -(C₀₋₅ alkylene)-CO-O-(C₁₋₅ alkyl), -(C₀₋₅ alkylene)-O-CO-(C₁₋₅ alkyl), -(C₀₋₅ alkylene)-CO-NH₂, -(C₀₋₅ alkylene)-CO-NH(C₁₋₅ alkyl), -(C₀₋₅ alkylene)-CO-N(C₁₋₅ alkyl)(C₁₋₅ alkyl), -(C₀₋₅ alkylene)-NH-CO-(C₁₋₅ alkyl), -(C₀₋₅ alkylene)-N(C₁₋₅ alkyl)-CO-(C₁₋₅, alkyl), -(C₀₋₅ alkylene)-SO₂-NH₂, -(C₀₋₅ alkylene)-SO₂-NH(C₁₋₅ alkyl), -(C₀₋₅ alkylene)-SO₂-N(C₁₋₅ alkyl)(C₁₋₅ alkyl), -(C₀₋₅ alkylene)-NH-SO2-(C₁₋₅ alkyl), -(C₀₋₅ alkylene)-N(C₁₋₅ alkyl)-SO₂(C₁₋₅ alkyl), -(C₀₋₅ alkylene)-SO-(C₁₋₅ alkyl), -(C₀₋₅ alkylene)-SO₂(C₁₋₅ alkyl), -(C₀₋₅ alkylene)-carbocyclyl, and -(C₀₋₅ alkylene)-heterocyclyl,
   wherein the carbocyclyl moiety in said -(C₀₋₅ alkylene)-carbocyclyl and the heterocyclyl moiety in said -(C₀₋₅ alkylene)-heterocyclyl are each optionally substituted with one or more R², wherein the C₀₋₅ alkylene moiety in said -(C₀₋₅ alkylene)-carbocyclyl and the C₀₋₅ alkylene moiety in said -(C₀₋₅ alkylene)-heterocyclyl are each optionally substituted with one or more R³, and further wherein one or more -CH₂- units comprised in the C₀₋₅ alkylene moiety in said -(C₀₋₅ alkylene)-carbocyclyl or in the C₀₋₅ alkylene moiety in said -(C₀₋₅ alkylene)-heterocyclyl are each optionally replaced by a group R⁴;
   wherein each R² is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), -SO-(C₁₋₅ alkyl), and -SO₂-(C₁₋₅ alkyl);
   wherein each R³ is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO2-(C₁₋₅ alkyl), -SO-(C₁₋₅ alkyl), and -SO₂-(C₁₋₅ alkyl);
   wherein each R⁴ is independently selected from -O-, -CO-, -CO-O-, -O-CO-, -NH-, -N(C₁₋₅ alkyl)-, -NH-CO-, -N(C₁₋₅ alkyl)-CO-, -CO-NH-, -CO-N(C₁₋₅ alkyl)-, -S-, -SO-, -SO₂-, -SO₂-NH-, -SO₂-N(C₁₋₅ alkyl)-, -NH-SO₂-, and -N(C₁₋₅ alkyl)-SO₂-;
   wherein one -OH in each polyphenol is optionally replaced by a group -O-(C₁₋₅ alkylene)-COOH, and wherein each polyphenol is covalently bound to the peptide via an amide linkage or an ester linkage.
7. The polyphenol-peptide conjugate of any one of items 1 to 6, wherein each polyphenol is independently selected from quercetin, quercetagetin, fisetin, galangin, gossypetin, herbacetin, kaempferol, morin, myricetin, robinetin, 5-O-methylmyricetin, annulatin, ayanin, axillarin, azaleatin, brickellin, centaureidin, chrysosplenetin, combretol, ermanin, eupatolitin, eupalitin, europetin, isorhamnetin, jaceidin, kaempferide, kumatakenin, laricitrin, natsudaidain, ombuin, pachypodol, patuletin, retusin, mearnsetin, rhamnazin, rhamnetin, santin, spinacetin, syringetin, tamarixetin, noricaritin, karanjachromene, karanjin, pyrocatechol, resorcinol, pyrogallol, phloroglucinol, catechin, epicatechin, catechin gallate, epicatechin gallate, gallocatechin, epigallocatechin, gallocatechin gallate, epigallocatechin gallate, taxifolin, afzelechin, fisetinidol, guibourtinidol, mesquitol, oritin, robinetinidol, meciadanol, and ourateacatechin,
   wherein one -OH in each polyphenol is optionally replaced by a group -O-(C₁₋₅ alkylene)-COOH, and wherein each polyphenol is covalently bound to the peptide via an amide linkage or an ester linkage.
8. The polyphenol-peptide conjugate of any one of items 1 to 7, wherein each polyphenol is 7-(carboxymethoxy)quercetin which is covalently bound to the peptide via a carboxamide linkage formed from the carboxy group of said 7-(carboxymethoxy)quercetin and an amino group of the peptide.
9. The polyphenol-peptide conjugate of any one of items 1 to 8, wherein said conjugate contains a single polyphenol which is covalently bound to the peptide via a carboxamide linkage formed from a carboxy group of the polyphenol and the N-terminal α-amino group of the peptide.
10. The polyphenol-peptide conjugate of any one of items 1 to 9, wherein said conjugate contains at least one cargo, and wherein each of the at least one cargo is independently selected from a peptide/protein, a nucleic acid, a lipid, a sugar, and an active pharmaceutical ingredient.
11. A silica particle comprising a polyphenol-peptide conjugate as defined in any one of items 1 to 10, wherein said polyphenol-peptide conjugate is encapsulated by a silica shell.
12. The silica particle of item 11, wherein said silica particle is obtainable by subjecting an aqueous solution of the polyphenol-peptide conjugate to silicic acid and thereby induce silica precipitation to obtain particles containing the polyphenol-peptide conjugate encapsulated by a silica shell.
13. The silica particle of item 11 or 12 for use as a medicament.
14. A pharmaceutical composition comprising the silica particle of item 11 or 12 and optionally a pharmaceutically acceptable excipient.
15. A method of delivering a cargo into the nucleus of a cell, the method comprising contacting the cell with a silica particle as defined in item 11 or 12.
16. Use of a silica particle as defined in item 11 or 12 as a nuclear-targeted delivery vehicle.
17. Use of a silica particle as defined in item 11 or 12 for nuclear-targeted gene or drug delivery.

The invention is also described by the following illustrative figures. The appended figures show:

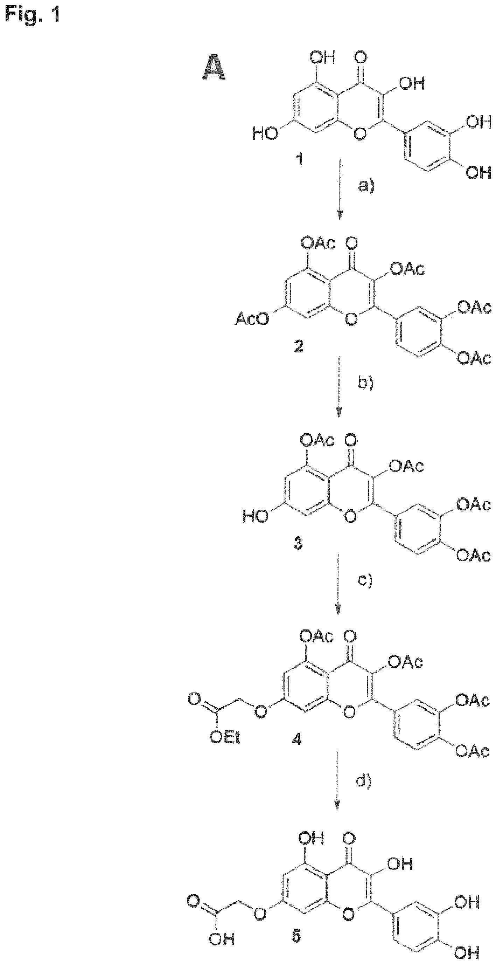

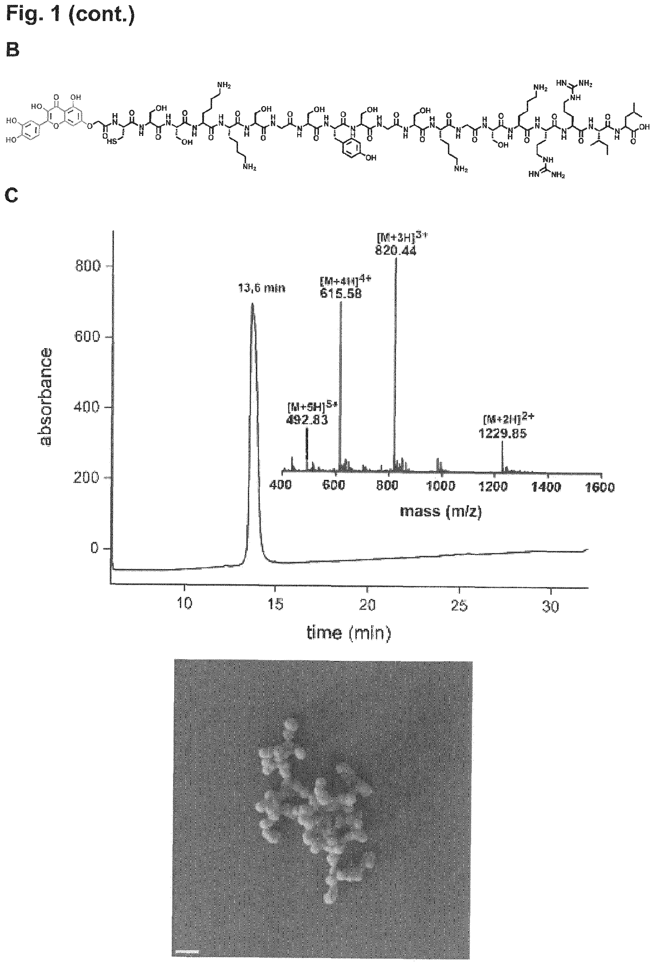

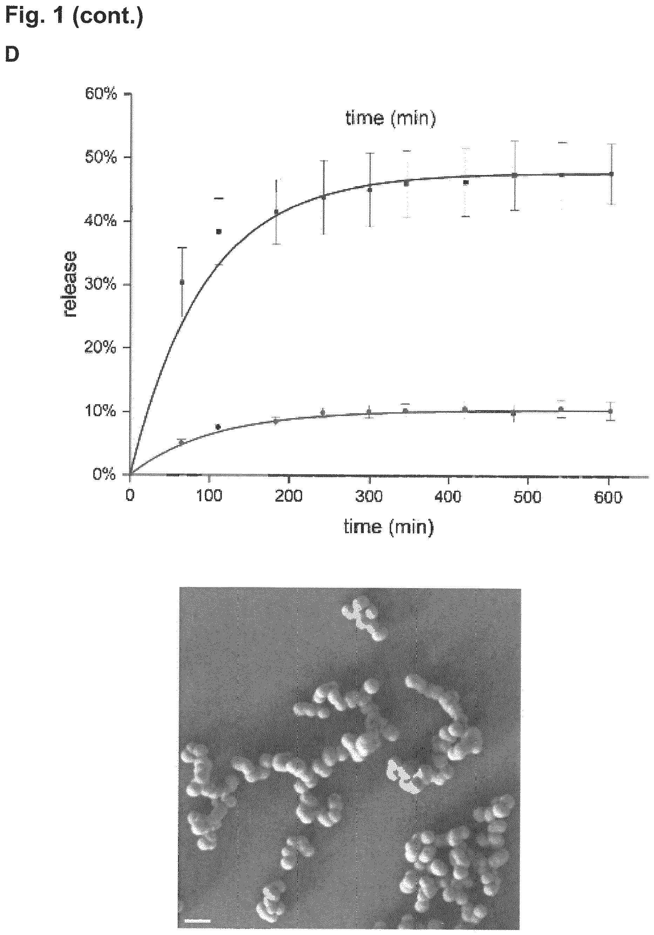

**Figure 1****:** Quercetin-R5 conjugate. (**A**) Synthesis of modified quercetin (11 % overall yield). Conditions: a) Ac₂O, pyridine, 70 °C; b) imidazole, PhSH, NMP, 0 °C - rt; c) DMF, Cs₂CO₃, ethyl iodoacetate, 0 °C - rt; d) acetone, HCl, reflux. (**B**) Quercetin modified R5 peptide. (**C**) Analytical data of quercetin-R5 conjugate (RP-HPLC, ESI-MS) and SEM picture of resulting silica particles. (**D**) Release of quercetin-R5 conjugate from silica particles followed by absorbance of the supernatant at 375 nm at pH 7 (bottom) and at pH 4 (top) with SEM of silica particles after release of quercetin-peptide conjugate. Scale bars: 1 µm. See Example 1.
**Figure 2****:** Uptake of R5-peptide containing silica particles (SiPs) into HT-29 cells was visualized by live cell fluorescence measurements with fluorescein-labeled R5 SiPs at t = 0 (**A**) and t = 3 h (**B**). See Example 2.
**Figure 3**: Analysis of quercetin-R5 SiP uptake into HT-29 cells by live cell fluorescence imaging (cell membrane is depicted in white). Pictures are showing the time course of uptake (**A**) 1 min; (**B**) 60 min; (**C**) 120 min; (**D**) 180 min. Appearance of the 3D reconstruction and of the cross sections SURFACE level and CENTRAL level of HT-29 cells 1 min after the application of SiPs (**E**) and at the end of the incubation (180 min. **F**). See Example 2.
**Figure 4****:** HPLC-MS analysis of conjugate released from the silica particles. (**A**) chromatogram, (**B**) mass spectrum (calc. M([M+2H]²⁺) = 1230.1). See Example 1.
**Figure 5****:** Spectra of quercetin-R5 conjugate. (**A**) absorbance, (**B**) excitation spectrum (540 nm emission), (**C**) emission spectrum (380 nm excitation), (**D**) emission spectrum (440 nm excitation). See Example 2.

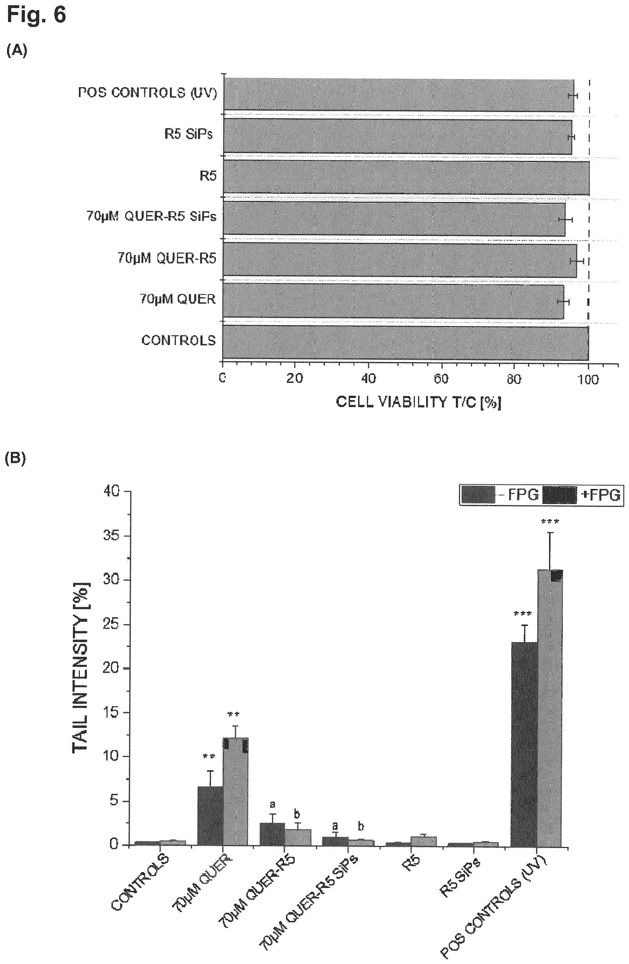

**Figure 6**: Impact of quercetin-R5 SiPs on DNA integrity in HT-29 cells. (**A**) Cell viability check measured in parallel to the comet assay measured by Tripan Blue exclusion test. (**B**) Tail intensity [%] measured during comet assay in presence (gray) or absence (black) of the formamidopyrimidine DNA-glycosylase (FPG) for the detection of oxidatively damaged DNA bases. Data are expressed as mean ±S.E. of n > 3 independent experiments measured in technical duplicates. * indicates significant difference in comparison to controls (**p < 0.01 and ***p < 0.001). Letters indicate significant differences in comparison to quercetin alone (a: incubation without FPG; b: incubation with FPG p < 0.05, one way ANOVA with Fisher test). See Example 2.
**Figure 7****:** Characterization of the cytotoxic potential of the R5 SiPs (particles) of the R5 peptide after sonication or suspension. (**A**) WST-1 measurement of the mitochondrial activity. (**B**) SRB measurements of the total cell protein content. See Example 2.
**Figure 8****:** Concentration-dependent detection of quercetin uptake by live cell imaging in HT-29 cells (cell membrane in white). See Example 2.
**Figure 9****:** Live cell imaging of the uptake of quercetin-R5 SiPs and interaction with the cell membrane. Active engulfment of the particles indicated with arrows. See Example 2.

The invention will now be described by reference to the following examples which are merely illustrative and are not to be construed as a limitation of the scope of the present invention.

### EXAMPLES

### Materials and methods (for Examples 1 and 2)

### Coupling to the R5 peptide

The 20 amino acid R5 peptide was synthesized on Wang resin using Fmoc-based SPPS. It was either done manually or on a CEM Liberty Blue peptide synthesizer (Matthews, NC). Coupling of the modified quercetin (2.5 equivalents) was performed on resin using 1.97 equivalents of HOBt and 1.95 equivalents of DIC for activation. The reaction was allowed to proceed overnight in the absence of light. The resin-bound product was dried before cleaving it with a standard cleavage cocktail (92.5 % TFA, 2.5 % H₂O, 5 % TiS). The cleaved conjugate was then purified by RP-HPLC using a C18 semiprep column. Purity of the conjugate was confirmed by analytical RP-HPLC and ESI-MS.

### Quercetin-R5 spectra

Spectra of the conjugate were recorded at 1 mg/mL concentration in 50 mM potassium phosphate buffer (pH 7). The most efficient fluorescence is observed when exciting at 440 nm and detecting at 540 nm wavelength. Absorbance was measured using a Thermo Scientific NanoDrop 2000c spectrophotometer. Excitation and emission spectra were collected using a Horiba Scientific FluoroMax-4 spectrofluorometer with 5 nm slit width.

### Silica particle generation

The nanoparticles were prepared from a 1 mg/mL solution of either the R5 peptide or the conjugate in 50 mM potassium phosphate buffer (pH 7) which was incubated overnight at room temperature. To 90 µL of the solution, 10 µL of silicic acid (freshly prepared from 960 µL of 1 mM hydrochloric acid and 40 µL of TMOS) were added. The mixture was vortexed and the precipitation allowed to proceed for 30 min, before separating the particles by centrifugation in a table-top centrifuge at 17,000 g for 5 min.

To create fluorescein-labeled silica particles, a solution of 0.1 mg ce386 in 250 µL 50 mM potassium phosphate buffer (pH 7) was incubated for 3 h. 10 µL of this solution were then added to the peptide solution in addition to the silicic acid before precipitation of the particles. Particles were washed three times with 100 µl water (Milli-Q) before further use.

### Scanning Electron Microscopy

For SEM imaging, the silica particles precipitated from 100 µL reaction volume were resuspended in 500 µL of water. 5 µL of the resuspended particles were spotted onto a cell culture coverslip, dried and sputtered with 5 nm of gold. Samples were then imaged on a Zeiss Supra 55 VP electron microscope using 5 kV accelerating voltage.

### HT-29 cells cultivation and toxicological characterization

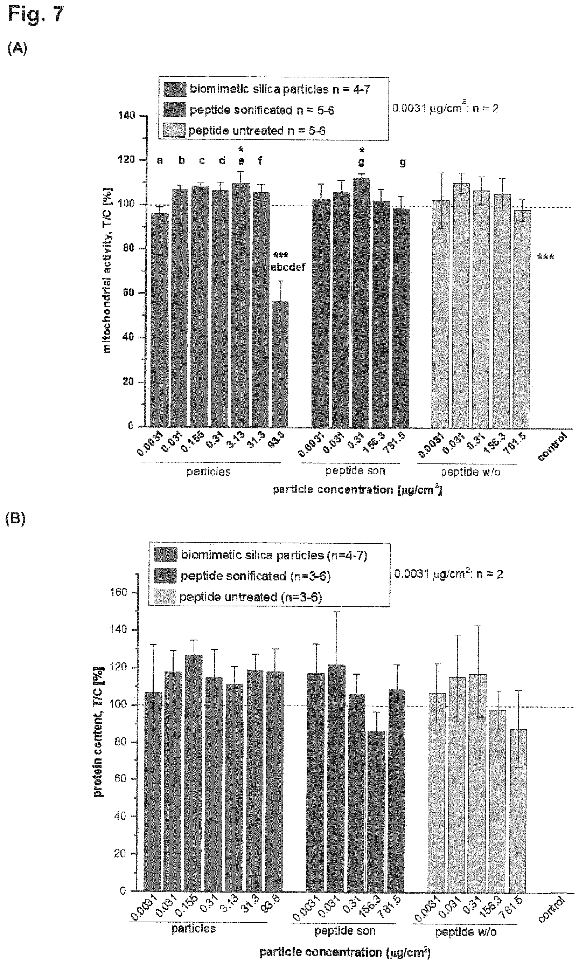

Human colon adenocarcinoma cells HT-29 (ATCC) were cultivated and used according to the specification of the supplier. Accordingly, cells were cultivated in humidified incubators at 37 °C and 5 % CO₂ in DMEM medium supplemented with 10 % fetal bovine serum (FBS) and 1 % penicillin/streptomycin (P/S, 50 U/ml). Cell culture media and supplements were purchased from GIBCO Invitrogen (Karlsruhe, Germany), Sigma-Aldrich Chemie GmbH (Munich, Germany) and disposables from Sarstedt AG&Co (Nuembrecht, Germany) and Ibidi (Martinsried, Germany). Cell viability presented in Figure 7 was measured with the Cell Proliferation Reagent WST-1 for mitochondrial activity (Roche Diagnostics GmbH, Mannheim, Germany; abs. 450 nm and reference wavelength 650 nm) or with sulforhodamine B assay for protein content (absorbance 570 nm) with a Victor³V 1420 Multilabel Counter Plate Reader (Perkin Elmer, Waltham, USA). Assays were performed according to the specification of the supplier as previously described in detail (Wittig A et al., Nanomaterials (Basel), 2017, 7(1), pii: E18; Del Favero G et al., Toxicol Lett, 2018, 295: 424-37). Genotoxic potential of quercetin-R5 SiPs was assessed by single-cell gel electrophoresis ("comet") assay as previously reported (Aichinger G et al., Mol Nutr Food Res, 2017, 61(2); Aichinger G et al., Toxicol Lett, 2018, 284: 136-42).

### Live cell imaging

Moreover, in order to verify the kinetic of the uptake of the quercetin-R5 SiPs in HT-29 cells, as well as if the uptake was an active process or consequence of an alteration of the permeability of the cell membrane, detailed live cell imaging experiments were performed. To this aim cells were stained with CellMask™ Deep Red Plasma membrane Stain (1:1000 dilution, white) and imaging was performed in Live Cell Imaging Solution (all from Molecular Probes, Life Technologies, Thermo Fisher Scientific, Waltham, USA). For the live cell imaging experiments a confocal LSM microscope Zeiss 710 equipped with ELYRA PS. 1 was used equipped with a Plan Apochromat 63X/1.4 oil objective.

### Example 1: Synthesis of a silica-encapsulated polyphenol-peptide conjugate (quercetin-coupled R5 peptide)

For introducing a carboxylic acid linker into quercetin without affecting its function, the inventors chose to address the 7-hydroxy group, as also illustrated in Figure 1A. This was achieved by first completely acetylating quercetin with an excess of acetic anhydride in pyridine at 70 °C. It was then regioselectively deprotected with imidazole and thiophenol in N-Methyl-2-pyrrolidone (NMP) as described by Kim et al. to give the 7-O-monodeacetylated product (Kim et al., J Med Chem, 2014, 57(17): 7216-33). Subsequently the carboxylic acid linker was introduced by addition of ethyl iodoacetate. The fully protected intermediate **3** (see Figure 1A) was then deprotected using hydrochloric acid in refluxing acetone as described by Mattarei et al. (11% yield over 4 steps; Mattarei et al., Molecules, 2010, 15(7): 4722-36).

Coupling to the 20mer R5 peptide (i.e., CSSKKSGSYSGSKGSKRRIL; SEQ ID NO: 1; see Figure 1B) was performed on resin prior to cleavage and purification. The cleaved conjugate was then purified by HPLC (see Figure 1C).

The resulting quercetin-R5 conjugate was dissolved at 1 mg/ml concentration in phosphate buffer at pH 7 and freshly generated silicic acid was added to give highly homogeneous silica particles with a peptide loading of >95% (ratio of peptide in the silica particles vs. peptide in solution, meaning that after precipitation, only up to 5 % fluorescence remained in the supernatant of the precipitation solution). Particles were separated by centrifugation, washed and imaged by scanning electron microscopy (SEM, see Figure 1C). The obtained particles were spherical with a diameter of approximately 400 nm.

Release of the conjugate from the silica particles in 50 mM potassium phosphate buffer at neutral pH and pH 4 was followed by UV-VIS spectroscopy, based on the absorbance of quercetin at 375 nm. Here, a pH-dependent release could be observed. At pH 4, 45% of the quercetin-R5 conjugate was found in the supernatant after 5 h (see Figure 1D), whereas at neutral pH only 10% were released after 5 h and no further increase in released conjugate was observed.

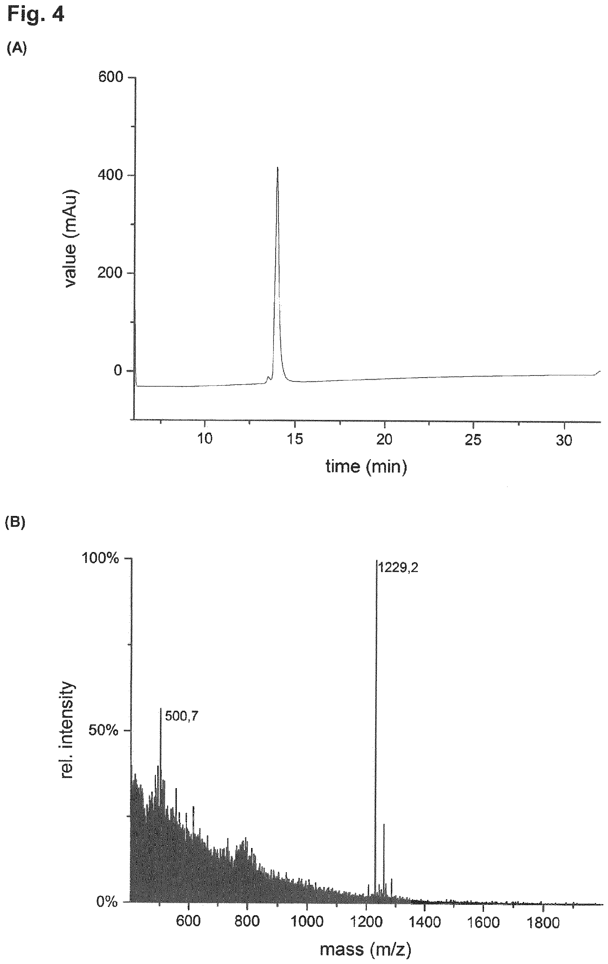

Therefore, quercetin R5 containing silica particles (SiPs) were incubated in aqueous buffer at pH 7.4 before incubation with cells for 5 h to remove loosely associated quercetin-R5. To investigate the morphology of the particles after release of a large fraction of the conjugate, a sample of the particles was incubated for 4.5 h in 50 mM phosphate buffer at pH 4. The particles were then imaged by SEM showing that they retained their spherical shape (see Figure 1D). The supernatant of the particles after 10 h incubation at pH 4 was also analyzed by LC-MS and clearly indicated that the released conjugate was fully intact (see Figure 4).

### Example 2: Biological evaluation of cellular uptake and localization of silica particles comprising a polyphenol-peptide conjugate in human cells

To test uptake of R5-based silica particles, human colon adenocarcinoma cells HT-29 were cultivated and incubated with R5-silica particles not containing quercetin. Scanning transmission electron microscopy (STEM) experiments were performed to analyze intact cells and to locate SiPs. The biomimetic R5-based SiPs not containing quercetin appeared to have affinity for the cellular membrane already after 1 h of incubation with HT-29 cells. Accordingly, after 24 h of incubation massive accumulation of SiPs in the intracellular compartment was observed.

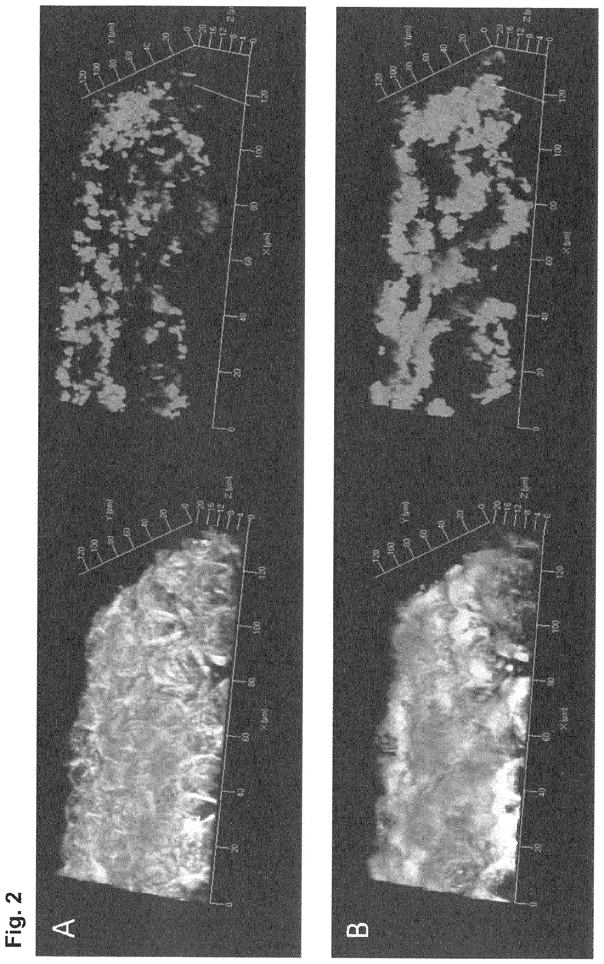

Once it was verified that the biomimetic SiPs can enter the intracellular compartment, a fluorescent variant was used in combination with live cell imaging to follow the kinetic of uptake at the cellular level. The fluorescein-labeled silane ce386 was incorporated into SiPs at a ratio of 1 to 20 to the R5 peptide. Similarly, a progressive uptake was observed that led to accumulation of the fluorescent SiPs in the cytoplasm of HT-29 cells after 3 h (see Figures 2A and 2B). Parallel cytotoxicity measurement revealed no toxicity from the biomimetic SiPs in HT-29 cells (see Figure 7A: mitochondrial activity WST-1; and Figure 7B: protein content SRB1 assay).

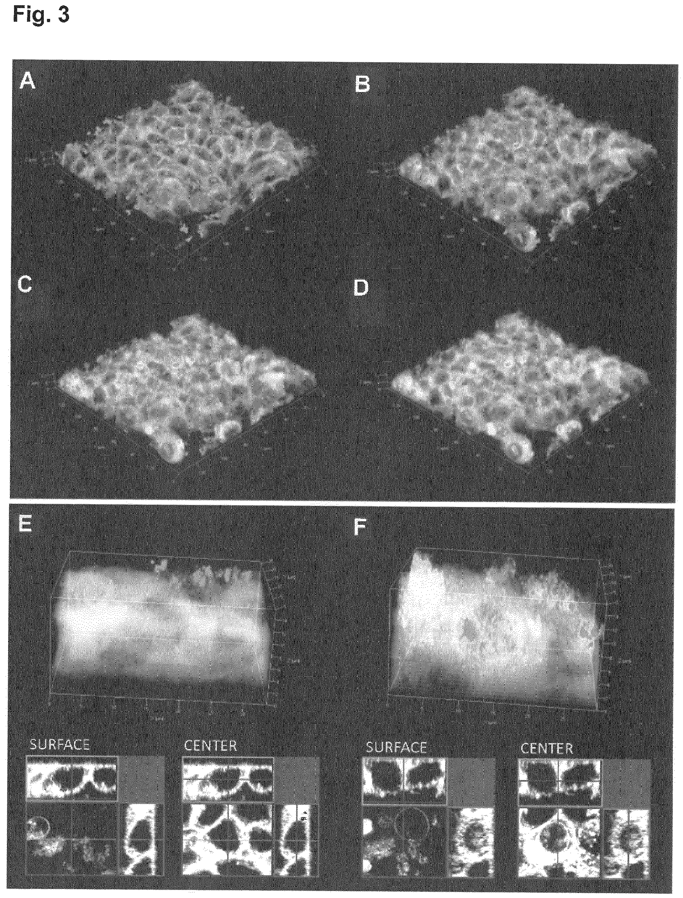

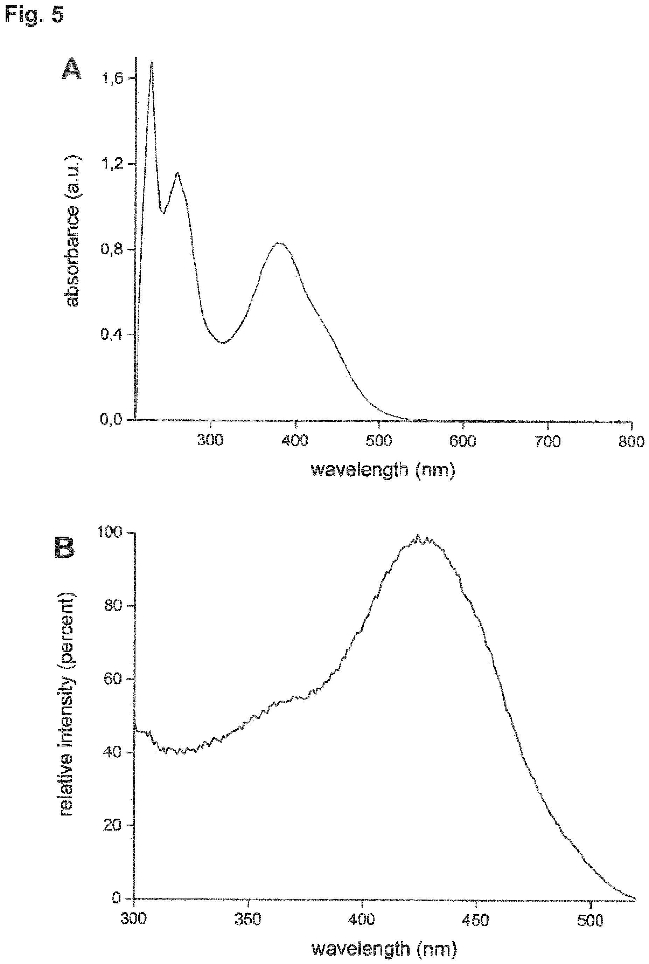

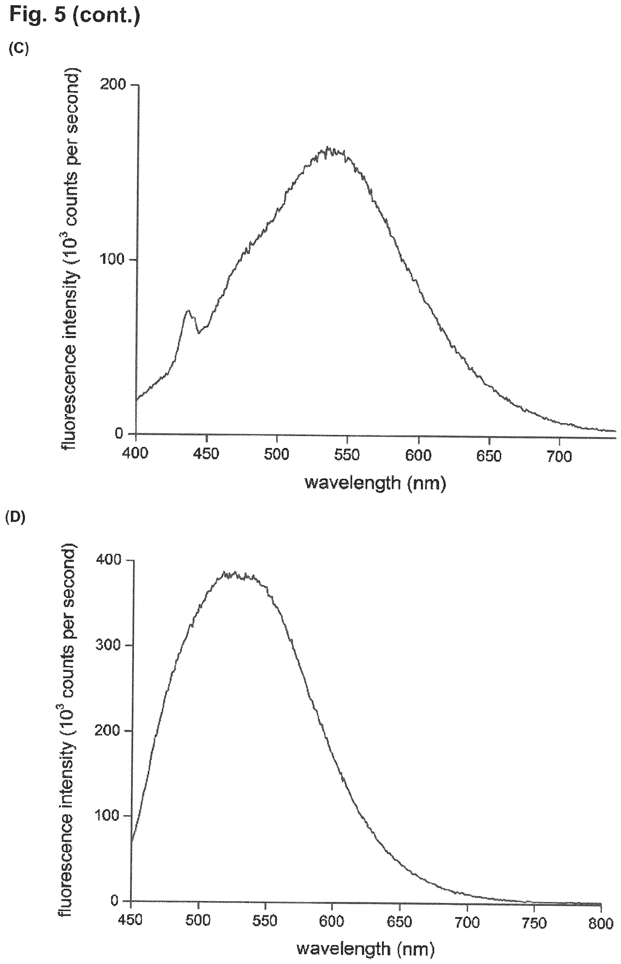

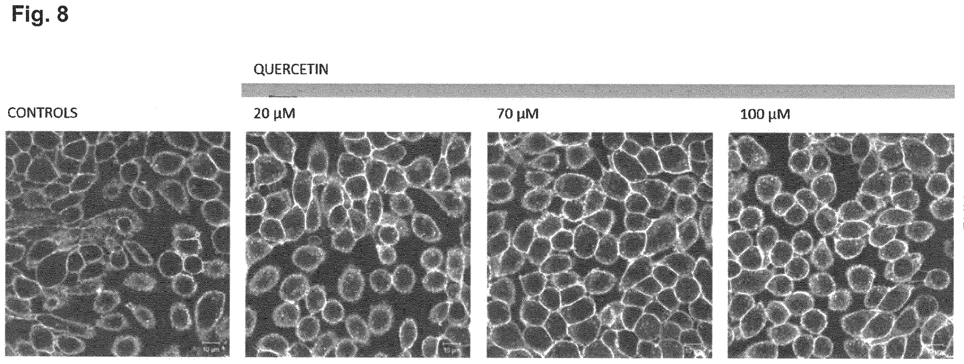

After demonstrating uptake of R5-SiPs into HT-29 cells without any cytotoxic effects on cell viability and DNA integrity, the inventors moved on to quercetin-R5-based SiPs. These SiPs containing quercetin-R5 conjugate (prepared as described in Example 1) behaved similarly to the R5- and fluorescein-R5-SiPs described above. Interestingly, it was noticed that the intrinsic fluorescence of the quercetin-R5 peptide, even when incorporated in SiPs, was sufficient for live cell imaging (see Figure 5: fluorescence spectrum of quercetin-R5; and Figure 8: concentration-dependent quercetin-associated fluorescence in HT-29 cells). This property of the quercetin-R5 conjugate allowed the inventors to study uptake without any need for additional labelling, which also excludes any undesired effects of labels on the uptake and targeting mechanisms. Here, uptake into HT-29 cells was followed by live cell imaging and proved that already after 3 h a major fraction of quercetin-R5 SiPs was found in cells (see Figures 3A to 3D).

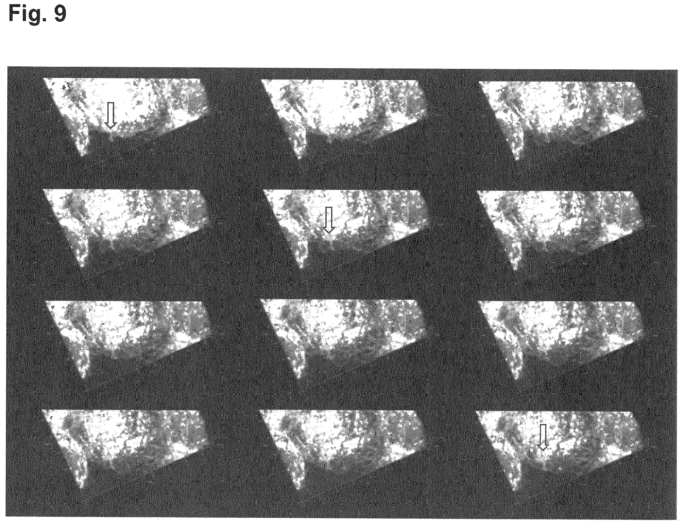

In order to verify if the uptake of quercetin-R5-SiPs was an active process or a consequence of an alteration of the permeability of the cell membrane, respective live cell imaging experiments were performed (see Figure 9). Confocal live imaging microscopy allowed to follow the uptake of quercetin-R5 SiPs and the subsequent interaction with the plasma membrane (see Figure 9). Accordingly, uptake of the quercetin-R5 SiPs occurred through active engulfment of the particles followed by recovery of the membrane surface to its original integrity, thus indicating the SiPs uptake to be an active process and not a secondary effect of the particles sustained by a loss of membrane integrity.

Moreover, the quercetin-modified SiPs accumulated inside the nucleus (see Figures 3E and 3F as well as the cross sections of the cell surface central section), a behavior not observed for the other R5-SiPs investigated here and rarely for silica (nano-)particles tested in other studies (AbouAitah K et al., Oncotarget, 2018, 9(41): 26466-90; Xiong L et al., Small, 2015, 11(44): 5919-26; and Hemmerich PH et al., PLoS One, 2013, 8(4): e62018). To control if this targeting effect was accompanied by a biological effect, comet assay (single cell gel electrophoresis) experiments were performed with the quercetin-R5 SiPs in comparison to the corresponding concentration of quercetin (70 µM), quercetin-R5 and R5 SiPs. Interestingly, binding of quercetin to SiPs abolished the genotoxic effect of the unconjugated compound (see Figure 6B).

It has thus been demonstrated that the silica-encapsulated polyphenol-peptide conjugate according to the present invention is efficiently taken up into human cells, without any detectable effects on cell viability and DNA integrity, and accumulates inside the cell nucleus. This nuclear targeting effect is achieved only if the conjugate is silica-encapsulated and contains both the polyphenol and the peptide. The silica-encapsulated polyphenol-peptide conjugate provided herein has thus been found to be highly effective in promoting cellular uptake and nuclear targeting, and can hence be used as a vehicle for nuclear-targeted gene or drug delivery. In particular, by attaching cargo molecules to the peptide component of the silica-encapsulated polyphenol-peptide conjugate, the conjugate of the present invention allows to efficiently deliver any such cargo molecules into cells and to release them inside the cell nucleus.

## Claims

1. A polyphenol-peptide conjugate consisting of:
- a peptide,
wherein said peptide comprises the amino acid sequence Arg-Arg-X¹-Leu, wherein X¹ refers to one or two hydrophobic amino acids which are each independently selected from lie, Leu, Val, Phe, Trp, and Met;
- at least one polyphenol,
wherein each polyphenol is covalently bound to the peptide;
- and optionally at least one cargo,
wherein each cargo, if present, is covalently bound to the peptide.

2. The polyphenol-peptide conjugate of claim 1, wherein the peptide comprises the amino acid sequence Arg-Arg-X¹-Leu, wherein X¹ refers to one hydrophobic amino acid selected from lie, Leu, and Val.

3. The polyphenol-peptide conjugate of claim 1 or 2, wherein the peptide comprises the amino acid sequence Arg-Arg-Ile-Leu.

4. The polyphenol-peptide conjugate of any one of claims 1 to 3, wherein the peptide consists of about 10 to about 40 amino acid residues, preferably of about 15 to about 30 amino acid residues, more preferably of about 20 amino acid residues.

5. The polyphenol-peptide conjugate of any one of claims 1 to 4, wherein the peptide comprises or consists of the amino acid sequence CSSKKSGSYSGSKGSKRRIL.

6. The polyphenol-peptide conjugate of any one of claims 1 to 5, wherein each of said at least one polyphenol is independently an arene or heteroarene, wherein said arene or said heteroarene is substituted with two or more -OH and is optionally further substituted with one or more R¹;
wherein each R¹ is independently selected from C₁₋₅ alkyl, C₂₋₅ alkenyl, C₂₋₅ alkynyl, -(C₀₋₅ alkylene)-OH, -(C₀₋₅ alkylene)-O(C₁₋₅ alkyl), -(C₀₋₅ alkylene)-O(C₁₋₅ alkylene)-OH, -(C₀₋₅ alkylene)-O(C₁₋₅ alkylene)-O(C₁₋₅ alkyl), -(C₀₋₅ alkylene)-SH, -(C₀₋₅ alkylene)-S(C₁₋₅ alkyl), -(C₀₋₅ alkylene)-S(C₁₋₅ alkylene)-SH, -(C₀₋₅ alkylene)-S(C₁₋₅ alkylene)-S(C₁₋₅ alkyl), -(C₀₋₅ alkylene)-NH₂, -(C₀₋₅ alkylene)-NH(C₁₋₅ alkyl), -(C₀₋₅ alkylene)-N(C₁₋₅ alkyl)(C₁₋₅ alkyl), -(C₀₋₅ alkylene)-halogen, -(C₀₋₅ alkylene)-(C₁₋₅ haloalkyl), -(C₀₋₅ alkylene)-O-(C₁₋₅ haloalkyl), -(C₀₋₅ alkylene)-CN, -(C₀₋₅ alkylene)-NO₂, -(C₀₋₅ alkylene)-CHO, -(C₀₋₅ alkylene)-CO-(C₁₋₅ alkyl), -(C₀₋₅ alkylene)-COOH, -(C₀₋₅ alkylene)-CO-O-(C₁₋₅ alkyl), -(C₀₋₅ alkylene)-O-CO-(C₁₋₅ alkyl), -(C₀₋₅ alkylene)-CO-NH₂, -(C₀₋₅ alkylene)-CO-NH(C₁₋₅ alkyl), -(C₀₋₅ alkylene)-CO-N(C₁₋₅ atkyl)(C₁₋₅ alkyl), -(C₀₋₅ alkylene)-NH-CO-(C₁₋₅ alkyl), -(C₀₋₅ alkylene)-N(C₁₋₅ alkyl)-CO-(C₁₋₅ alkyl), -(C₀₋₅ alkylene)-SO₂-NH₂, -(C₀₋₅ alkylene)-SO₂-NH(C₁₋₅ alkyl), -(C₀₋₅ alkylene)-SO₂-N(C₁₋₅ alkyl)(C₁₋₅ alkyl), -(C₀₋₅ alkylene)-NH-SO₂-(C₁₋₅ alkyl), -(C₀₋₅ alkylene)-N(C₁₋₅ alkyl)-SO2-(C₁₋₅ alkyl), -(C₀₋₅ alkylene)-SO-(C₁₋₅ alkyl), -(C₀₋₅ alkylene)-SO₂-(C₁₋₅ alkyl), -(C₀₋₅ alkylene)-carbocyclyl, and -(C₀₋₅ alkylene)-heterocyclyl,
wherein the carbocyclyl moiety in said -(C₀₋₅ alkylene)-carbocyclyl and the heterocyclyl moiety in said -(C₀₋₅ alkylene)-heterocyclyl are each optionally substituted with one or more R², wherein the C₀₋₅ alkylene moiety in said -(C₀₋₅ alkylene)-carbocyclyl and the C₀₋₅ alkylene moiety in said -(C₀₋₅ alkylene)-heterocyclyl are each optionally substituted with one or more R³, and further wherein one or more -CH₂- units comprised in the C₀₋₅ alkylene moiety in said -(C₀₋₅ alkylene)-carbocyclyl or in the C₀₋₅ alkylene moiety in said -(C₀₋₅ alkylene)-heterocyclyl are each optionally replaced by a group R⁴;
wherein each R² is independently selected from C₁₋₅ alkyl, C₂₋₅ alkenyl, C₂₋₅ alkynyl, -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -SO₂-NH₂, -SO₂-NH(C₁₋₅ alkyl), -SO₂N(C₁₋₅ alkyl)(C₁₋₅ alkyl), -NH-SO₂-(C₁₋₅ alkyl), -N(C₁₋₅ alkyl)-SO₂(C₁₋₅ alkyl), alkyl), and -SO₂-(C₁₋₅ alkyl); wherein each R³ is independently selected from -OH, -O(C₁₋₅ alkyl), -O(C₁₋₅ alkylene)-OH, -O(C₁₋₅ alkylene)-O(C₁₋₅ alkyl), -SH, -S(C₁₋₅ alkyl), -S(C₁₋₅ alkylene)-SH, -S(C₁₋₅ alkylene)-S(C₁₋₅ alkyl), -NH₂, -NH(C₁₋₅ alkyl), -N(C₁₋₅ alkyl)(C₁₋₅ alkyl), halogen, C₁₋₅ haloalkyl, -O-(C₁₋₅ haloalkyl), -CF₃, -CN, -NO₂, -CHO, -CO-(C₁₋₅ alkyl), -COOH, -CO-O-(C₁₋₅ alkyl), -O-CO-(C₁₋₅ alkyl), -CO-NH₂, -CO-NH(C₁₋₅ alkyl), -CO-N(C₁₋₅ alkyl)(C₁₋₅ alkyl), -NH-CO-(C₁₋₅ alkyl), -N(C₁₋₅ alkyl)-CO-(C₁₋₅ alkyl), -SO₂-NH₂, -SO₂-NH(C₁₋₅ alkyl), -SO₂-N(C₁₋₅ alkyl)(C₁₋₅ alkyl), -NH-SO₂-(C₁₋₅ alkyl), -N(C₁₋₅ alkyl)-SO₂-(C₁₋₅ alkyl), alkyl), and -SO₂-(C₁₋₅ alkyl);
wherein each R⁴ is independently selected from -O-, -CO-, -CO-O-, -O-CO-, -NH-, -N(C₁₋₅ alkyl)-, -NH-CO-, -N(C₁₋₅ alkyl)-CO-, -CO-NH-, -CO-N(C₁₋₅ alkyl)-, -S-, -SO-, -SO₂-, -SO₂-NH-, -SO₂-N(C₁₋₅ alkyl)-, -NH-SO₂-, and -N(C₁₋₅ alkyl)-SO₂-;
wherein one -OH in each polyphenol is optionally replaced by a group -O-(C₁₋₅ alkylene)-COOH, and wherein each polyphenol is covalently bound to the peptide via an amide linkage or an ester linkage.

7. The polyphenol-peptide conjugate of any one of claims 1 to 6, wherein each polyphenol is independently selected from quercetin, quercetagetin, fisetin, galangin, gossypetin, herbacetin, kaempferol, morin, myricetin, robinetin, 5-O-methylmyricetin, annulatin, ayanin, axillarin, azaleatin, brickellin, centaureidin, chrysosplenetin, combretol, ermanin, eupatolitin, eupalitin, europetin, isorhamnetin, jaceidin, kaempferide, kumatakenin, laricitrin, natsudaidain, ombuin, pachypodol, patuletin, retusin, mearnsetin, rhamnazin, rhamnetin, santin, spinacetin, syringetin, tamarixetin, noricaritin, karanjachromene, karanjin, pyrocatechol, resorcinol, pyrogallol, phloroglucinol, catechin, epicatechin, catechin gallate, epicatechin gallate, gallocatechin, epigallocatechin, gallocatechin gallate, epigallocatechin gallate, taxifolin, afzelechin, fisetinidol, guibourtinidol, mesquitol, oritin, robinetinidol, meciadanol, and ourateacatechin,
wherein one -OH in each polyphenol is optionally replaced by a group -O-(C₁₋₅ alkylene)-COOH, and wherein each polyphenol is covalently bound to the peptide via an amide linkage or an ester linkage.

8. The polyphenol-peptide conjugate of any one of claims 1 to 7, wherein each polyphenol is 7-(carboxymethoxy)quercetin which is covalently bound to the peptide via a carboxamide linkage formed from the carboxy group of said 7-(carboxymethoxy)quercetin and an amino group of the peptide.

9. The polyphenol-peptide conjugate of any one of claims 1 to 8, wherein said conjugate contains a single polyphenol which is covalently bound to the peptide via a carboxamide linkage formed from a carboxy group of the polyphenol and the N-terminal α-amino group of the peptide.

10. The polyphenol-peptide conjugate of any one of claims 1 to 9, wherein said conjugate contains at least one cargo, and wherein each of the at least one cargo is independently selected from a peptide/protein, a nucleic acid, a lipid, a sugar, and an active pharmaceutical ingredient.

11. A silica particle comprising a polyphenol-peptide conjugate as defined in any one of claims 1 to 10, wherein said polyphenol-peptide conjugate is encapsulated by a silica shell.

12. The silica particle of claim 11, wherein said silica particle is obtainable by subjecting an aqueous solution of the polyphenol-peptide conjugate to silicic acid and thereby induce silica precipitation to obtain particles containing the polyphenol-peptide conjugate encapsulated by a silica shell.

13. A pharmaceutical composition comprising the silica particle of claim 11 or 12 and optionally a pharmaceutically acceptable excipient.

14. An *in vitro* method of delivering a cargo into the nucleus of a cell, the method comprising contacting the cell with a silica particle as defined in claim 11 or 12.

15. Use of a silica particle as defined in claim 11 or 12 as a nuclear-targeted delivery vehicle, preferably for nuclear-targeted gene or drug delivery.


SEQUENCE LISTING

<110> Universitaet Wien

<120> Polyphenol-peptide conjugates for nuclear-targeted delivery

<130> AB2031 EP

<160> 14

<170> BiSSAP 1.3.6

<210> 1
<211> 20
<212> PRT
<213> Cylindrotheca fusiformis


<220>
<223> R5 peptide

<400> 1
Cys Ser Ser Lys Lys Ser Gly Ser Tyr Ser Gly Ser Lys Gly Ser Lys
1               5                   10                  15
Arg Arg Ile Leu
            20

<210> 2
<211> 5
<212> PRT
<213> Artificial Sequence


<220>
<223> Peptide

<220>
<221> VARIANT
<222> 3
<223> X = absent or Ile, Leu, Val, Phe, Trp or Met

<220>
<221> VARIANT
<222> 4
<223> X = Ile, Leu, Val, Phe, Trp or Met

<400> 2
Arg Arg Xaa Xaa Leu
1               5

<210> 3
<211> 4
<212> PRT
<213> Artificial Sequence


<220>
<223> Peptide

<220>
<221> VARIANT
<222> 3
<223> X = Ile, Leu or Val

<400> 3
Arg Arg Xaa Leu
1

<210> 4
<211> 4
<212> PRT
<213> Artificial Sequence

<220>
<223> Peptide

<400> 4
Arg Arg Ile Leu
1

<210> 5
<211> 5
<212> PRT
<213> Artificial Sequence

<220>
<223> Peptide

<400> 5
Arg Arg Ile Ile Leu
1               5

<210> 6
<211> 5
<212> PRT
<213> Artificial Sequence

<220>
<223> Peptide

<400> 6
Arg Arg Ile Leu Leu
1               5

<210> 7
<211> 5
<212> PRT
<213> Artificial Sequence

<220>
<223> Peptide

<400> 7
Arg Arg Ile Val Leu
1               5

<210> 8
<211> 5
<212> PRT
<213> Artificial Sequence

<220>
<223> Peptide

<400> 8
Arg Arg Leu Ile Leu
1               5

<210> 9
<211> 5
<212> PRT
<213> Artificial Sequence


<220>
<223> Peptide

<400> 9
Arg Arg Leu Leu Leu
1               5

<210> 10
<211> 5
<212> PRT
<213> Artificial Sequence


<220>
<223> Peptide

<400> 10
Arg Arg Leu Val Leu
1               5

<210> 11
<211> 5
<212> PRT
<213> Artificial Sequence


<220>
<223> Peptide

<400> 11
Arg Arg Val Ile Leu
1               5

<210> 12
<211> 5
<212> PRT
<213> Artificial Sequence


<220>
<223> Peptide

<400> 12
Arg Arg Val Leu Leu
1               5

<210> 13
<211> 5
<212> PRT
<213> Artificial Sequence

<220>
<223> Peptide

<400> 13
Arg Arg Val Val Leu
1               5

<210> 14
<211> 5
<212> PRT
<213> Artificial Sequence


<220>
<223> Peptide

<400> 14
Lys Arg Arg Ile Leu
1               5